(19) **Europäisches Patentamt / European Patent Office / Office européen des brevets**

(11) **EP 4 087 465 B1**

(12) # EUROPEAN PATENT SPECIFICATION

(45) Date of publication and mention of the grant of the patent:
**06.05.2026 Bulletin 2026/19**

(21) Application number: **21738592.1**

(22) Date of filing: **04.01.2021**

(51) International Patent Classification (IPC):
*A61B 3/113* *(2006.01)*    *A61B 5/103* *(2006.01)*
*A61B 5/00* *(2006.01)*    *A61B 5/11* *(2006.01)*
*A61B 5/398* *(2021.01)*    *A61B 5/297* *(2021.01)*

(52) Cooperative Patent Classification (CPC):
**A61B 3/113; A61B 5/1114; A61B 5/1126; A61B 5/297; A61B 5/398; A61B 5/4863; A61B 5/6814;** A61B 5/103; A61B 2560/0242; A61B 2562/0219

(86) International application number:
**PCT/US2021/012076**

(87) International publication number:
**WO 2021/141850 (15.07.2021 Gazette 2021/28)**

(54) **WEARABLE NYSTAGMUS DETECTION DEVICES**

AM KÖRPER TRAGBARE NYSTAGMUSDETEKTIONSVORRICHTUNGEN

DISPOSITIFS DE DÉTECTION DE NYSTAGMUS POUVANT ÊTRE PORTÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2020 US 202062957563 P**

(43) Date of publication of application:
**16.11.2022 Bulletin 2022/46**

(60) Divisional application:
**25217136.8 / 4 691 368**

(73) Proprietor: **The Board of Trustees of the Leland Stanford**
**Junior University**
**Stanford, CA 94305-2038 (US)**

(72) Inventors:
• **RESSMEYER, Ryan Kazuo**
  **Mercer Island, Washington 98040 (US)**
• **SANTA MARIA, Peter Luke**
  **Menlo Park, California 94062 (US)**
• **KUO, Po Hung**
  **Redwood City, California 94063 (US)**
• **SILVERNAGEL, Michael Paul**
  **Redwood City, California 94063 (US)**
• **POON, Ada Shuk Yan**
  **Redwood City, California 94065 (US)**
• **STEENERSON, Kristen K.**
  **Palo Alto, California 94304 (US)**
• **KARGOTICH, Stephen**
  **Menlo Park, California 94025 (US)**
• **FAN, Danyang**
  **Palo Alto, California 94303 (US)**
• **DHULDHOYA, Jay**
  **Chino Hills, California 91709 (US)**

(74) Representative: **Arnold & Siedsma**
**Bezuidenhoutseweg 57**
**2594 AC The Hague (NL)**

(56) References cited:
**EP-A1- 3 338 623**    **WO-A1-93/02616**
**DE-A1- 102017 117 053**    **GB-A- 2 574 580**
**US-A- 5 726 916**    **US-A1- 2011 015 469**
**US-A1- 2017 000 329**    **US-A1- 2018 008 141**
**US-A1- 2018 049 663**    **US-A1- 2018 160 968**

**(Cont. next page)**

EP 4 087 465 B1

- **PHILLIPS JOHN S. ET AL: "An investigation into the diagnostic accuracy, reliability, acceptability and safety of a novel device for Continuous Ambulatory Vestibular Assessment (CAVA)", SCIENTIFIC REPORTS, vol. 9, no. 1, 1 December 2019 (2019-12-01), pages 10452, XP055840663, DOI: 10.1038/s41598-019-46970-7**
- **PHILLIPS ET AL.: "An investigation into the diagnostic accuracy, reliability, acceptability and safety of a novel device for Continuous Ambulatory Vestibular Assessment (CAVA", SCIENTIFIC REPORTS, vol. 9, no. 1, December 2019 (2019-12-01), pages 10452, XP055840663, Retrieved from the Internet <URL:https://www.nature.com/articles/s41598-019-46970-7>**
- **GOLPARVAR ET AL.: "Graphene Smart Textile-BasedWearable Eye Movement Sensor for Electro-Ocular Control and Interaction with Objects", JOURNAL OF THE ELECTROCHEMICAL SOCIETY, vol. 166, no. 9, 30 April 2019 (2019-04-30), pages B3185 - B3193, XP055840666, Retrieved from the Internet <URL:https://iopscience.iop.org/articte/10.1149/2.0241907jes/meta>**

## Description

### CROSS-REFERENCE TO RELATED APPLICATIONS

This application claims the benefit of U.S. Provisional Application No. 62/957,563, filed January 6, 2020.

### TECHNICAL FIELD

The subject matter described herein relates to wearable device with sensors to detect or derive corneo-retinal potential signals related to eye movement of a subject and circuitry operably coupled to the sensors.

### BACKGROUND

Nystagmus refers to characteristic eye movements that may arise in patients when they experience attacks of dizziness originating from underlying vestibular or neurological conditions. Such characteristic eye movements, nystagmus events, result from the neural connections between the inner ear and the eye, i.e., the vestibular ocular reflex, and generally do not occur when a patient is not experiencing dizziness. Nystagmus events can be characterized based on the nature of the eye movements exhibited by patients. Differentiating the types of eye movements may facilitate diagnosis of a patients' underlying vestibular or neurological conditions. For example, patients with benign paroxysmal positional vertigo tend to exhibit triggered nystagmus typically lasting less than about sixty seconds in at least three distinct directional patterns. Patients with Meniere's disease tend to exhibit unidirectional horizontally beating nystagmus lasting twenty minutes to twelve hours with then reversal of direction following the attack. Patients with vestibular migraines tend to exhibit combinations of vertical and horizontal nystagmus lasting up to several hours to days. Thus, even though patients with these different conditions may all express the common complaints of an attack of dizziness, examining the nature of nystagmus events may facilitate diagnosing the underlying vestibular or neurological conditions. Accurate diagnosis of these conditions is important, in part, because of the dramatic range of treatments for each condition, from a repositioning maneuver for benign paroxysmal positional vertigo, to trans-tympanic injections or surgery for Meniere's disease, to oral medications for vestibular migraines.

Traditionally, patients would have to visit a doctor's office or other clinical setting for detection of the different nystagmus events and diagnosis of the associated, underlying conditions. Currently, a common technique for doing so in a clinic is video nystagmography (VNG). VNG entails a patient wearing head-mounted goggles with infrared cameras that image and record eye movements of the patient during about two hours of testing in the clinic. Unfortunately, the diagnostic accuracy of VNG is generally poor since patients are not necessarily likely to experience a dizziness attack while undergoing testing in the clinic. Other limitations of VNG include unrepresentative and sub-physiologic measurements of vestibular function, delayed as well as limited accessing of VNG testing due to the need for bulky, stationary equipment and highly skilled technologists or audiologists, a high cost to insurance companies, limitations due to directly imaging and recording eye movements such as being unable to perform measurements when the eyes are closed, sensing artifacts from blinking, eye makeup or a structurally obtrusive eyelid architecture.

EP 338 623 AI discloses determining eye movement using piezo-electric sensors.

Thus, an alternative technique for monitoring eye movements so as to detect nystagmus events in patients has the potential to improve diagnoses and patient treatment as well as to reduce associated costs. The devices, systems, methods and kits described herein provide such techniques.

### BRIEF SUMMARY

The invention is defined by a device according to claim 1. Further embodiments are defined by dependent claims 2-11.

In one embodiment, the circuitry comprises an analog front end and a digital circuit.

In one embodiment, the analog front end comprises a noise filtering circuit and an amplifier circuit.

In one embodiment, the digital circuit comprises an analog-to-digital converter and a microcontroller.

In one embodiment, the digital circuit further comprises a digital signal processor.

In one embodiment, the first and second sensors are configured to measure a difference in electrical potential between a cornea and a retina of the subject.

In one embodiment, the first and second sensors are configured to measure electrical activity of muscles. In one embodiment, the first and second sensors are configured to measure the electrical activity of extraocular and facial muscles.

In one embodiment, the one or more first electrodes are positioned at one or more first locations and the one or more second electrodes are positioned at one or more second locations, wherein the one or more first and second locations are proximal to one eye of the subject.

In one embodiment, the one or more first electrodes and the one or more second electrodes are positioned asymmetrically with respect to horizontal and vertical axes that intersect a pupil.

In one embodiment, the one or more first electrodes and the one or more second electrodes are surface electrodes. In one embodiment, the sensors, electrodes or surface electrodes are dry electrodes. In another embodiment, the sensors, surface electrodes and/or electrodes are wet electrodes.

In one embodiment, the device is configured to monitor eye movement continuously. In one embodiment, the device is configured to monitor eye movement in near real time.

In one embodiment, the device further comprises a third sensor configured to sense head movement, position and/or orientation of the subject, wherein the circuitry is operably coupled to the third sensor and is further configured to detect head movement, position and/or orientation based on signals from the third sensor. In one embodiment, the device is configured to monitor eye movement based on the head movement, position and/or orientation. In another embodiment, the third sensor also functions as a trigger to activate the one or more electrodes on the device to initiate detection of signal associated with eye movement.

In one embodiment, the device comprising a third sensor is configured to monitor head movement, position and/or orientation along three axes. In one embodiment, the third sensor is an accelerometer. In one embodiment, the third sensor is an inertial mass unit, a gyroscope, an accelerometer or a magnetometer. In one embodiment the third sensor is a combination of these. In one embodiment, the third sensor is configured to detect three-dimensional head position, movement and/or orientation. In another embodiment, the third sensor is configured to detect direction, speed of movement and/or acceleration associated with head movement.

In one embodiment, the device further comprises a storage component; wherein the storage component is operably coupled to the circuitry; and the circuitry and the storage component are configured to record eye movement data and head movement, position and/or orientation data onto the storage component.

In one embodiment, the storage component is a removable memory card.

In one embodiment, the device further comprises a transmitter, wherein the transmitter is operably coupled to the circuitry; and the circuitry and the transmitter are configured to transmit eye movement data and head movement, position and/or orientation data.

In one embodiment, the transmitter is a wireless transmitter and the circuitry, and the wireless transmitter are configured to wirelessly transmit eye movement data and head movement, position and/or orientation data.

In one embodiment, the wireless transmitter is a wireless network interface controller.

In one embodiment, the wireless transmitter is a Bluetooth interface controller.

In one embodiment, the device is configured to transmit eye movement data and head movement, position and/or orientation data in near real time.

In one embodiment, the device further comprises a photosensor configured to sense ambient light, wherein the circuitry is operably coupled to the photosensor and is configured to detect ambient light based on signals from the photosensor.

In one embodiment, the device is configured to continuously monitor eye movement, head movement, position and/or orientation and ambient light. In one embodiment, the device is configured to monitor eye movement, head movement, position and/or orientation and ambient light in near real time.

In one embodiment, the first, second and third sensors and the circuitry are integrated onto a single substrate.

In one embodiment, the substrate is a printed circuit board.

In one embodiment, the first, second and third sensors, the circuitry and the storage component are integrated onto a single printed circuit board. In another embodiment, the first, second and optional third sensor are on a substrate separate from the electronic component and/or storage component.

In one embodiment, the first, second and third sensors, the circuitry and the wireless network interface controller are integrated onto a single printed circuit board.

In one embodiment, the first, second and third sensors, the circuitry and the photosensor are integrated onto a single printed circuit board.

In one embodiment, the device comprises a single wearable patch.

In one embodiment, the device comprises more than one wearable patches.

In one embodiment, the wearable patch is configured to be adhered to a facial location of the subject.

In one embodiment, the wearable patch is configured to be attached to a facial location of the subject using non-adhesive material.

In one embodiment, the wearable patch is flexible so as to be fitted to a facial location of the subject.

In one embodiment, the wearable patch is, adjustable, fitted or fitable, or moldable so as to be form-fitted to a facial location of the subject.

In one embodiment, the wearable patch is configured to be torn so as to be fitted to a facial location of the subject. In another embodiment, the wearable patch is configured to be assembled so as to be fitted to a facial location on a subject. In another embodiment, the wearable patch is provided a separate components comprised of the single unitary substrate and one or more electrodes, sensors, and electronic components, and the provided components are assembled to tailor fit a facial location on a subject. After use, the components are removed from the substrate and from the face of the user, for reuse or for disposable.

In one embodiment, the device is waterproof.

In another aspect, a system for detecting eye movements of a subject comprises a wearable device as described herein, and a software application installable onto a mobile device comprising a processor operably coupled to a memory that includes instructions stored thereon for interfacing with the device as well as an additional storage component. The wearable device and the mobile device are operably coupled such that data originating from the first and second sensors are accessible to the mobile device.

In one embodiment, the wearable device of the system further comprises a third sensor configured to sense head movement, position and/or orientation, wherein the circuitry is operably coupled to the third sensor and is further configured to detect head movement, position and/or orientation based on signals from the third sensor; and the wearable device and the mobile device are operably coupled such that data originating from the first, second and third sensors are accessible to the mobile device.

In one embodiment, the operable coupling between the device and the mobile device is a wireless connection.

In one embodiment, the processor, the memory and the instructions stored thereon are configured to apply an algorithm to the data for classifying different eye movements.

In one embodiment, the processor, the memory and the instructions stored thereon are configured to apply an algorithm to the data for distinguishing between horizontal, vertical and torsional eye movements.

In one embodiment, the processor, the memory and the instructions stored thereon are configured to apply an algorithm to the data to recognize one or more specific patterns of eye movement sensor data comprising one or more characteristic eye movements.

In one embodiment, the processor, the memory and the instructions stored thereon are configured to apply an algorithm to the data to recognize characteristic eye movements corresponding to neutral gaze, leftward gaze, rightward gaze, upward gaze and downward gaze.

In one embodiment, the processor, the memory and the instructions stored thereon are configured to apply an algorithm to the data to recognize characteristic eye movements corresponding a nystagmus event.

In one embodiment, the algorithm is configured to detect torsional eye movements.

In one embodiment, the algorithm is configured to distinguish between horizontal, vertical and torsional eye movements.

In one embodiment, the algorithm is configured to recognize one or more specific patterns of eye movement sensor data comprising one or more characteristic eye movements.

In one embodiment, characteristic eye movements that the algorithm is configured to recognize include neutral gaze, leftward gaze, rightward gaze, upward gaze and downward gaze.

In one embodiment, a characteristic eye movement that the algorithm is configured to recognize is a nystagmus event.

In one embodiment, the algorithm is configured to recognize a nystagmus event in near real time.

In one embodiment, the algorithm is configured to distinguish between characteristic eye movements of horizontal nystagmus events, vertical nystagmus events and torsional nystagmus events. In another embodiment, the algorithm is configured to detect a pattern of eye movement, including but not limited to jerk nystagmus, pendular nystagmus, congenital nystagmus, rebound nystagmus, positional nystagmus, gaze evoked and end gaze nystagmus. Such patterns of eye movement can occur spontaneously or can be triggered by a specific inducer. The algorithm in other embodiments is configured to detect smooth pursuit and its abnormal iterations and/or saccades and its abnormal iterations.

In one embodiment, the algorithm is configured to recognize characteristic eye movements of nystagmus events associated with benign paroxysmal positional vertigo.

In one embodiment, the algorithm is configured to recognize characteristic eye movements of nystagmus events associated with Menière's disease.

In one embodiment, the algorithm is configured to recognize characteristic eye movements of nystagmus events associated with vestibular neuritis.

In one embodiment, the algorithm applied to the data is a machine learning algorithm.

In one embodiment, the system is configured to recognize a nystagmus event in near real time.

In one embodiment, the system is configured to distinguish between characteristic eye movements corresponding to horizontal nystagmus events, vertical nystagmus events and torsional nystagmus events.

In one embodiment, the system is configured to recognize characteristic eye movements of nystagmus events associated with benign paroxysmal positioning vertigo.

In one embodiment, the system is configured to recognize characteristic eye movements of nystagmus events associated with Menière's disease.

In one embodiment, the system is configured to recognize characteristic eye movements of nystagmus events associated with vestibular neuritis.

In one embodiment, the processor, the memory and the instructions stored thereon are configured to record data originating from the first and second sensors onto the additional storage component.

In one embodiment, the processor, the memory and the instructions stored thereon are configured to record data originating from the first, second and third sensors onto the additional storage component.

In one embodiment, the processor, the memory and the instructions stored thereon are configured to record data originating from the first, second and third sensors and the photosensor onto the additional storage component.

In one embodiment, the mobile device further comprises a display; and the processor, the memory and the instructions stored thereon are configured to display a graphical representation of the data originating from the first and second sensors onto the display.

In one embodiment, the system is configured to display a graphical representation of the data originating from the first and second sensors in near real time.

In another aspect not forming part of the claimed invention, a method of detecting horizontal and vertical eye movements in a subject is provided. The method comprises sensing electrical activity of the eye at a first location on the subject, sensing electrical activity of the eye at a second location on the subject; and measuring electrical signals correlated with eye movement based on the electrical activity sensed at the first and second locations on the subject, wherein the first and second locations are on a single side of the subject's face.

In one embodiment, measuring electrical signals correlated with eye movement based on the electrical activity sensed at the first and second locations on the subject comprises measuring the difference in electrical potential between the subject's cornea and retina based on the electrical activity sensed at the first and second locations on the subject.

In one embodiment, measuring electrical signals correlated with eye movement based on the electrical activity sensed at the first and second locations on the subject comprises measuring electrical activity of muscles sensed at the first and second locations on the subject.

In one embodiment, the method is performed using a wearable device, the device comprising first and second sensors configured to sense eye movement of the subject; and circuitry operably coupled to the sensors and configured to detect horizontal and vertical eye movements based on signals from the first and second sensors.

In one embodiment, the method is performed using a system comprising a wearable device, the device comprising first and second sensors configured to sense eye movement of the subject; and a transmitter configured to transmit signals sensed by the first and second sensors to remote circuitry configured to receive signals transmitted by the transmitter and to detect horizontal and vertical eye movement based on signals from the first and second sensors; and a software application downloadable to or downloaded onto a mobile device comprising a processor operably coupled to a memory that includes instructions stored thereon for interfacing with the device as well as an additional storage component. The wearable device and the mobile device are operably coupled such that data originating from the first and second sensors are accessible to the mobile device.

In one embodiment, the method further comprises sensing orientation and/or acceleration of the head at a third location on the subject; and measuring the movement of the subject's head based on the acceleration sensed at the third location on the subject. In an embodiment, the sensor detects three dimensional head position and/or acceleration of head movement, including onset, speed and starting and finishing points of the head movement.

In one embodiment, the method is performed using a wearable device comprising first and second sensors configured to sense eye movement of the subject, and a third sensor configured to sense head movement of the subject; and circuitry operably coupled to the first, second and third sensors and configured to detect horizontal and vertical eye movements based on signals from the first and second sensors and head movement based on signals from the third sensor. In one embodiment, the method is performed using a system comprising a wearable device, the device comprising first and second sensors configured to sense eye movement of the subject, and a third sensor configured to sense head movement of the subject; and a transmitter configured to transmit signals sensed by the first, second and third sensors to remote circuitry configured to receive signals transmitted by the transmitter and to detect horizontal and vertical eye movement based on signals from the first and second sensors and to detect head movement based on signals from the third sensor. The system also comprises a software application that, in one embodiment, is downloadable to or downloaded onto a mobile device comprising a processor operably coupled to a memory that includes instructions stored thereon for interfacing with the device as well as an additional storage component. In other embodiments, the software application is stored on a server or a computer or non-mobile device. The wearable device and the mobile device or non-mobile device with the software application are operably coupled such that data originating from the first, second and third sensors are accessible to the mobile device or non-mobile device.

In one embodiment, the method further comprises sensing ambient light; and measuring ambient light based on the ambient light sensed.

In one embodiment, the method is performed using a wearable device, the device comprising first and second sensors configured to sense eye movement of the subject, a third sensor configured to sense head movement of the subject, a photosensor configured to sense ambient light; and circuitry operably coupled to the first, second and third sensors and the photosensor and configured to detect horizontal and vertical eye movements based on signals from the first and second sensors, head movement, position and/or orientation based on signals from the third sensor and ambient light based on signals from the photosensor.

In one embodiment, the method is performed using a system, the system comprising a wearable device, the device comprising first and second sensors configured to sense eye movement of the subject; a third sensor configured to sense head movement, position and/or orientation of the subject; a photosensor configured to sense ambient light; and a transmitter configured to transmit signals sensed by the first, second and third sensors and photosensor to remote circuitry configured to receive signals transmitted by the transmitter and to detect horizontal and vertical eye movement based on signals from the first and second sensors, head movement, position and/or orientation based on signals from the third sensor and ambient light based on signals from the photosensor. The system also comprises a software application downloadable to or downloaded onto a mobile device comprising a processor operably coupled to a memory that includes instructions stored thereon for interfacing with the device as well as an additional storage component. The software application comprises an algorithm for processing signal from the wearable device. The device and the mobile device are operably coupled such that data originating from the first, second and third sensors and the photosensor are accessible to the mobile device. In another embodiment, the software application is on a server, a computer or other non-mobile computing device. The wearable device and the mobile device or non-mobile computing device with the software application are in wireless communication to transmit data originating from the first, second and third sensors to the mobile device or non-mobile device for processing by the algorithm.

In another aspect, a kit for monitoring eye movement of a subject comprises a wearable device for monitoring eye movement of a subject and configured to be applied to a single side of a subject's face during use, as described herein. The device is further comprising packaging for the device.

In addition to the exemplary aspects and embodiments described above, further aspects and embodiments will become apparent by reference to the drawings and by study of the following descriptions.

Additional embodiments of the present devices, systems, methods and kits will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded from any embodiment of the present disclosure. Additional aspects and advantages of the present disclosure are set forth in the following description and claims, particularly when considered in conjunction with the accompanying examples and drawings.

## BRIEF DESCRIPTION OF THE FIGURES

FIGS. 1A-1B depict embodiments of a wearable device for sensing electrical activity associated with monocular corneo-retinal potential.
FIGS. 2A-2H are illustrations of a wearable device positioned unilaterally on a face of a user, according to embodiments.
FIG. 2I depicts electrode placement of devices in the prior art.
FIGS. 3A-3B depict circuitry of the wearable device

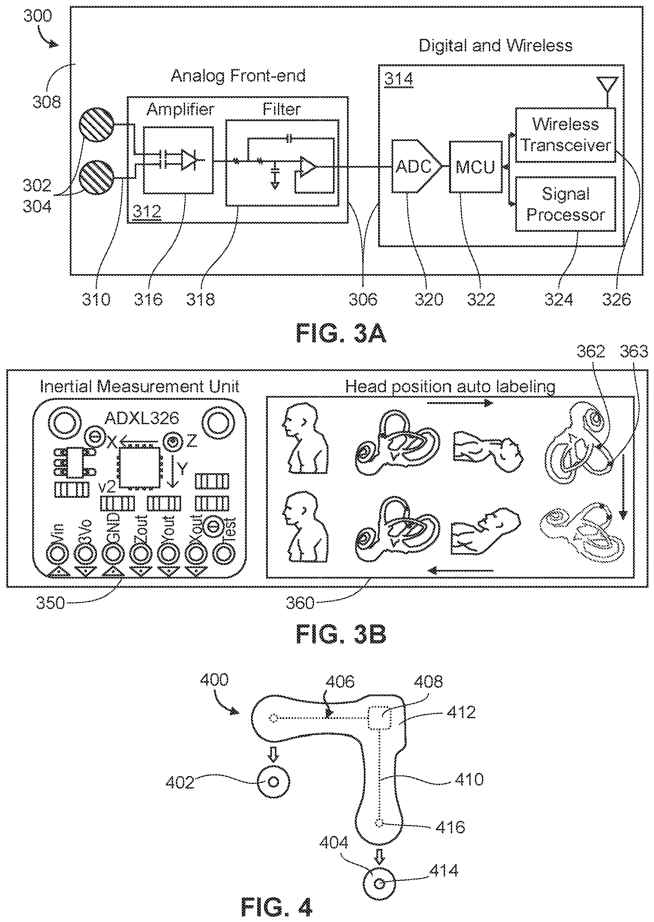

according to some embodiments, where the embodiment of FIG. 3B shows a sensor configured to sense head movement, position and/or orientation of a subject.
FIG. 4 is an illustration of a single wearable patch or single wearable device demonstrating one way for affixing it to a face of a user.

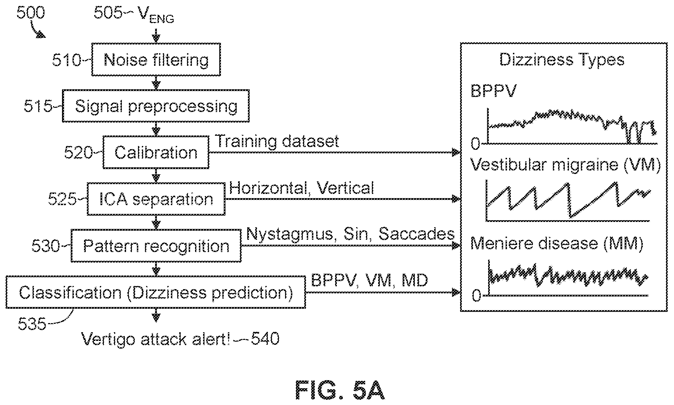

FIGS. 5A-5F illustrate a processing algorithm and its constituent steps that may be applied to a signal detected by a wearable device of a system, according to some embodiments.

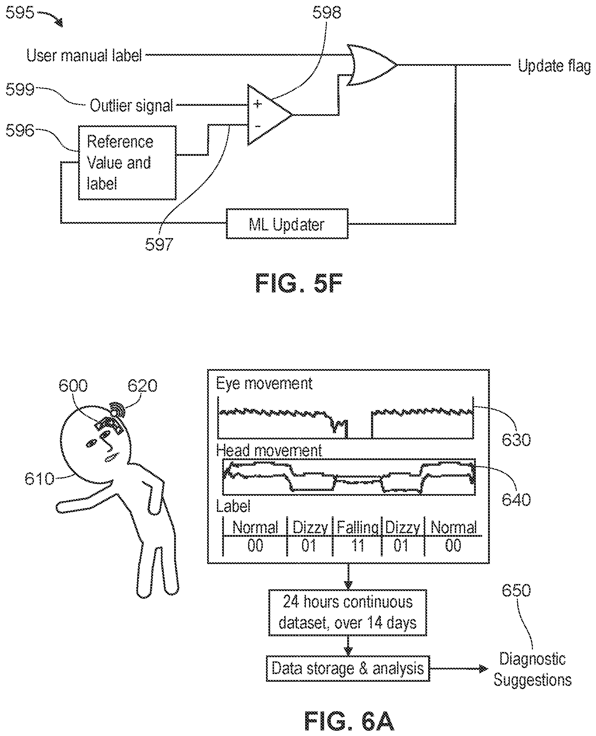

FIGS. 6A-6B illustrate pattern recognition and classification applied to signals detected by a system, according to some embodiments.

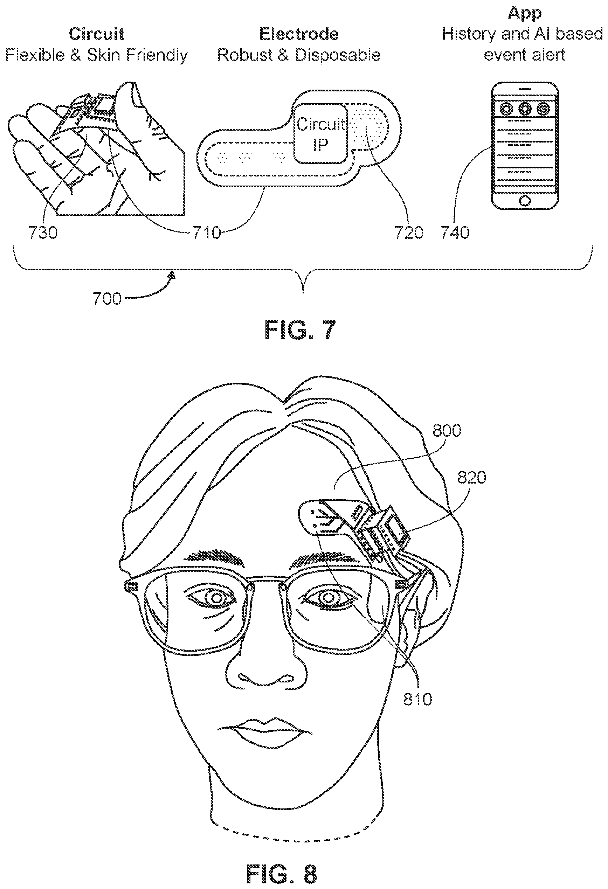

FIG. 7 provides a depiction of a system for detecting eye movements of a subject comprising a wearable device and a software application installable onto or installed on a mobile device, according to one embodiment.
FIG. 8 provides a depiction of a version of the wearable device, according to some embodiments.

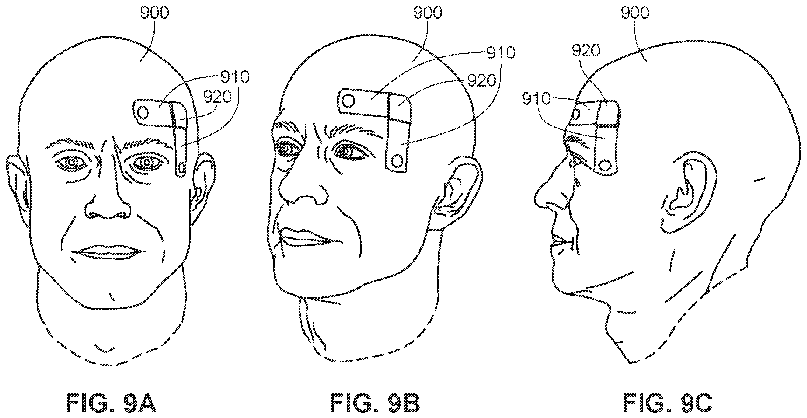

FIGS. 9A-9C illustrates embodiments of a wearable device.

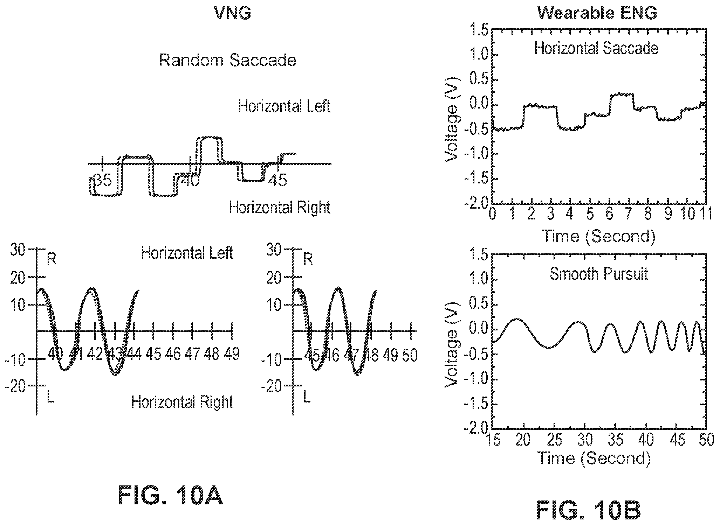

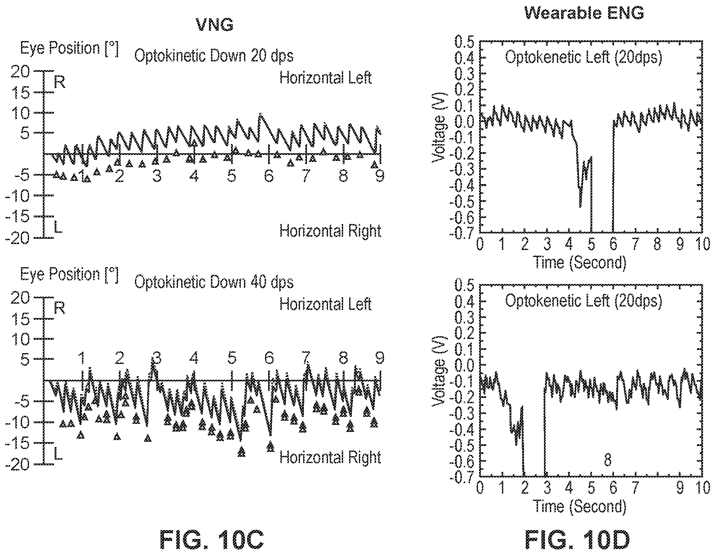

FIGS. 10A-10G present results of a comparison between the device similar to that depicted in FIG. 8 and a standard clinical diagnostic method, VNG goggles, under conditions of saccades, smooth pursuit and optokinetics.

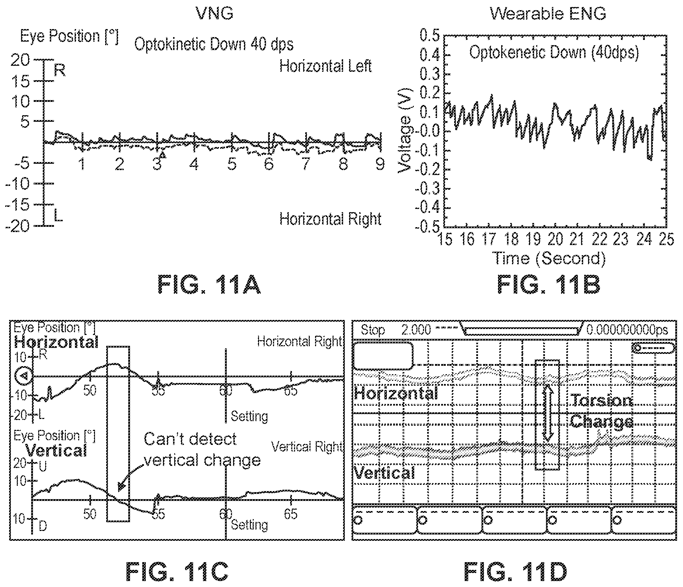

FIGS. 11A-11D presents results of a comparison between the device similar to that depicted in FIG. 8 and a standard clinical diagnostic method, VNG goggles, under conditions of downward viewing.

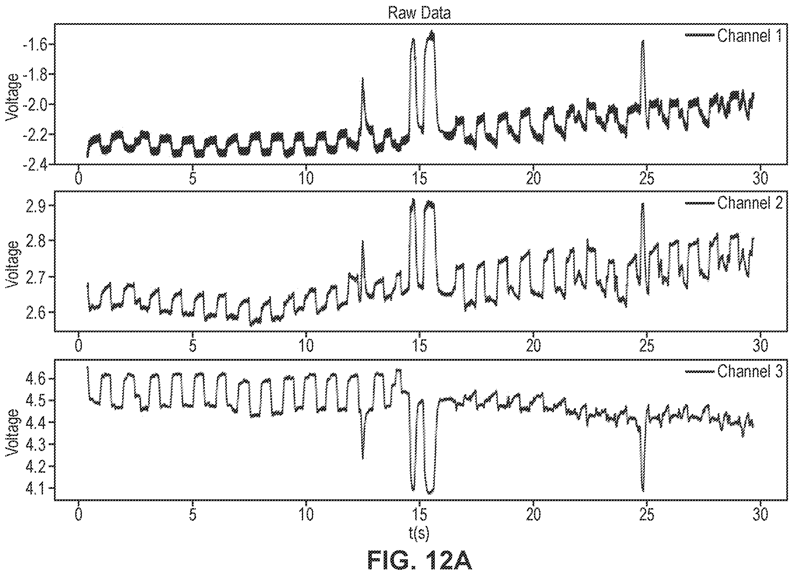

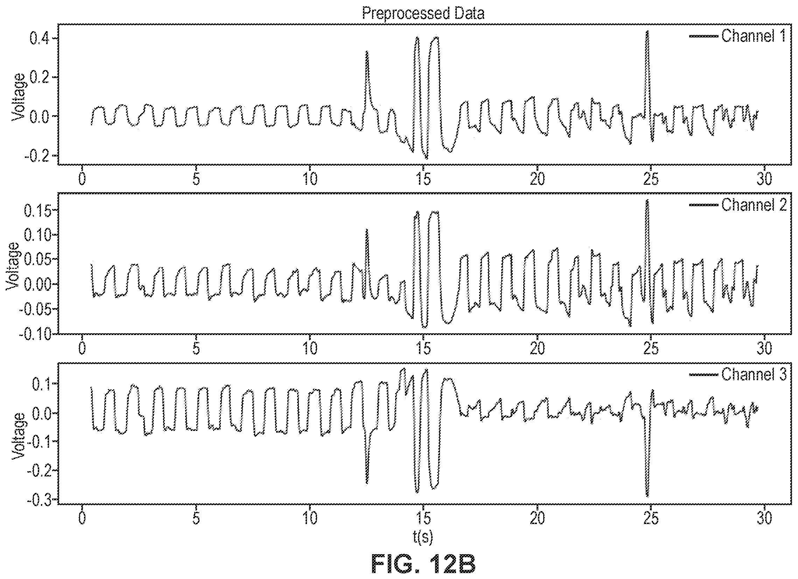

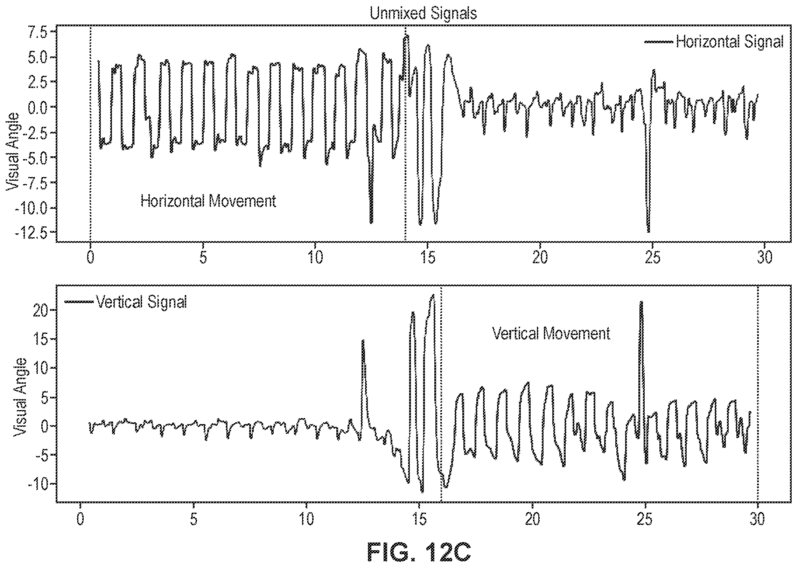

FIGS. 12A-12C show data from a wearable device with three electrodes, where FIG. 12A shows the raw data from signal detected by each electrode (upper panel, middle panel and lower panel), FIG. 12B shows the data after pre-processing of the raw signal from each electrode to remove noise and baseline drift (upper panel, middle panel and lower panel), and FIG. 12C shows the data after processing by an algorithm to discriminate signal associated with horizontal eye movement from signal associated with vertical eye movement.

## DETAILED DESCRIPTION

Before the devices, systems, kits and methods are described in greater detail, it is to be understood that they are not limited to particular embodiments described, as such may, of course, vary. It is also to be understood that the terminology used herein is for the purpose of describing particular embodiments only, and is not intended to be limiting, since the scope of the devices, systems, kits and methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements or use of a "negative" limitation.

Any recited method can be carried out in the order of events recited or in any other order which is logically possible.

I. Wearable Device and Systems Comprising the Device

Devices, systems, methods and kits for monitoring eye movement of a subject are provided. The devices, systems, methods and kits find use in a variety of different applications, for example, diagnosing conditions based on the presence of horizontal and/or vertical eye movement of a subject, such as subjects with benign paroxysmal positioning vertigo, Meniere's disease or vestibular migraines.

The device is comprised of a unitary substrate comprising a first electrode, a second electrode, and circuitry operably coupled to the first electrode and the second electrode. The unitary substrate is dimensioned for unilateral placement on a user's face to position the first electrode and the second electrode to detect electrical signals correlated with horizontal and vertical eye movement.

In another embodiment, the wearable device for monitoring eye movement of a subject comprises first and second sensors configured to sense eye movement of the subject, and circuitry operably coupled to the sensors and configured to detect horizontal and/or vertical eye movements based on signals from the first and second sensors. The device, in another embodiment, comprises a transmitter configured to transmit signals sensed by the first and second sensors to remote circuitry configured to receive signals transmitted by the transmitter and to detect horizontal and/or vertical eye movement based on signals from the first and second sensors. In another embodiment, the wearable device comprises first and second sensors configured to sense eye movement of a subject; and (i) circuitry operably coupled to the sensors and configured to detect horizontal and vertical eye movements based on signals from the sensors, and/or (ii) a transmitter configured to transmit data originating from the first and second sensors to remote circuitry configured to detect horizontal and vertical eye movements based on signals from the sensors. In these embodiments, each sensor may comprise a single electrode, two electrodes, three electrodes, four electrodes, or more.

As will be described in greater detail below, the wearable device functions as part of a system that is comprised of a wearable device and a software application on a mobile device. The software application may have an algorithm for analysis of data from the wearable device, and other features that will are described *infra*.

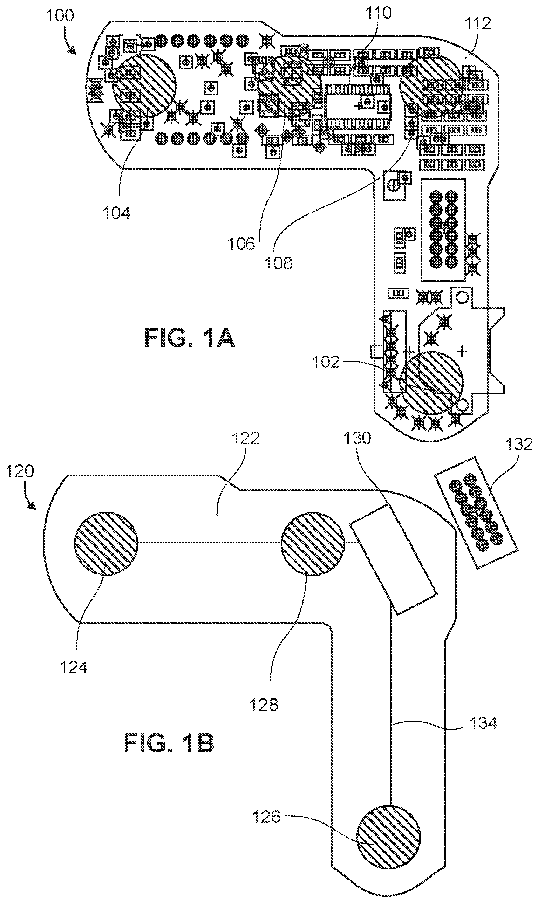

An embodiment of the wearable device is depicted in **FIG. 1A**. A device **100** is comprised of a first sensor **102** and a second sensor **104**. The embodiment also includes additional optional sensors, **106, 108.** The sensors, in this embodiment, comprise a single electrode; however, it is contemplated that a sensor may also comprise two or more electrodes. It is also contemplated that a sensor is a component other than an electrode; for example, in some embodiments the term sensor refers to an accelerometer, a gyroscope, a magnetometer or an inertial mass unit. Device **100** also comprises circuitry **110.** The sensors and the circuitry are integrated onto a single, unitary substrate **112.** The unitary substrate **112** in this embodiment is a printed circuit board, although other embodiments are possible and are contemplated. For example, **FIG. 1B** illustrates another embodiment of a wearable device **120** having a unitary substrate **122** on which a first electrode **124** and a second electrode **126,** and an optional sensor or third electrode **128,** are mounted, embedded or affixed. In one embodiment, the first, second, and/or optional sensor or third electrode are removably insertable onto or into the unitary substrate. A compartment **130** is positioned at least partially on unitary substrate **122** and is dimensioned to receive, and optionally release, an electronic component **132.** The electronic component may comprise a printed circuit board with electronics, described below. The electronic component is insertable, and preferably removably insertable, into the compartment. When inserted into the compartment, it is in electronic communication with the first electrode, the second electrode and the optional sensor or third electrode, on the substrate by other elements of the circuitry on the substrate, such as traces or leads **134.** In the embodiments of **FIGS. 1A-1B,** the first and second sensors, or first and second electrodes, and the circuitry are mounted into or onto - that is, are held together by - the substrate. The substrate is substantially an "L" shape, however, other geometries are possible. In this embodiment, the first and second sensors or electrodes are separated from each other on the substrate. The device or portions of the device can be reusable or disposable. For example, the substrate with integrated sensors can be reusable, the substrate can be disposable and have removably insertable sensors and/or electronic component that are reusable by insertion into a fresh, unused substrate.

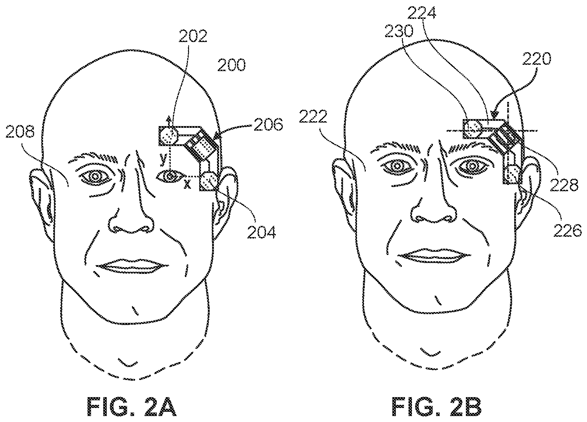

When the device is worn on the face of a subject, the locations of the first and second sensors or electrodes on the face of the subject are defined, at least in part, by the positions of the first and second sensors on the substrate. With reference to **FIGS. 2A-2B,** embodiments of wearable devices affixed unilaterally on a user's face are shown. In **FIG. 2A,** a device **200** is comprised of a first sensor **202,** a second senor **204** and circuitry **206.** Device **200** is configured to be applied to a single side of a face **208** of subject or a user. In the embodiment of **FIG. 2A,** first sensor **202** and second sensor **204** are positioned at first and second locations proximal to one eye of the subject. More particularly, when the device is affixed to the face, first sensor **202** is positioned asymmetrically with respect to a vertical axis that intersects the pupil of the eye when looking straight ahead, denoted by the dashed line indicated as "y". Second sensor **204** is positioned asymmetrically with respect to a horizontal axis that intersects the pupil of the eye when looking straight ahead, denoted by the dashed line indicated as "x." The asymmetric position of the sensors is evident by inspection of **FIG. 2A,** wherein first sensor **202** has a circular geometry with a center, and vertical axis y is asymmetric with respect to the center. Similarly, the asymmetric position of the second sensor **204,** which also has a circular geometry with a center, is evident because vertical axis x is asymmetric with respect to the center.

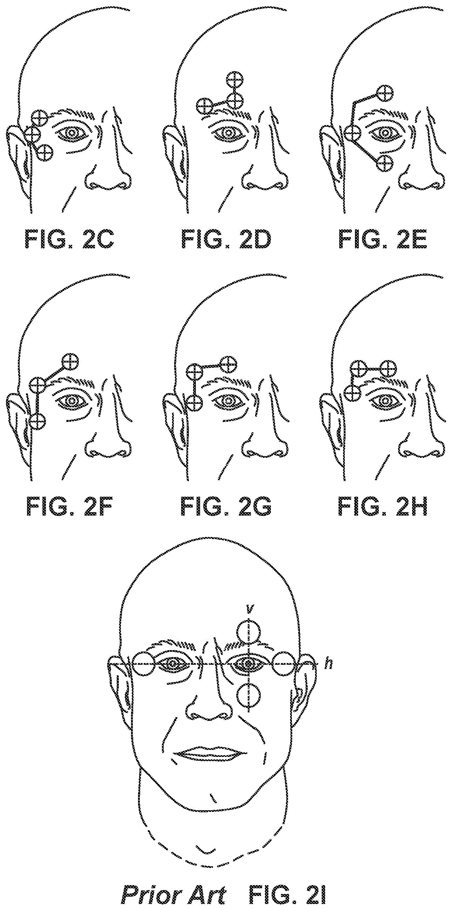

**FIG. 2B** depicts another embodiment of a device **220** positioned unilaterally on a face **222** of a subject. Device **220** has a single, unitary substrate **224** comprising a first electrode **226,** a second electrode **228,** and a sensor or electrode **230.** The device comprises circuitry operably coupled to the electrodes and/or sensors, where the circuitry, in this embodiment, includes an electronic component in the region of electrode **228.** The unitary substrate is dimensioned for unilateral placement on the user's face to position sensor **230** superior to a transverse (horizontal) plane passing through the inferior border of one of the right eye or the left eye, and electrode **226** temporally to a sagittal plane passing through the nasal border of the eye. In one embodiment, the first sensor is positioned on the substrate such that when placed on a user's face a midpoint of a plane through the first sensor is superior to a transverse (horizontal) plane passing through the inferior border of one of the right eye or the left eye, and the second sensor is positioned on the substrate such that when placed on a user's face a midpoint of a plane of the second sensor is positioned temporally to a sagittal plane passing through the nasal border or the inner corner of the eye. It will be appreciated that placement of the electrodes and/or sensors need not be along vertical and/or horizontal axes of the pupil, as shown in some of the configurations of FIGS. 2D-2G, discussed below.

The device in **FIG. 2B** shows an arrangement wherein one electrode is placed near an outside corner of one eye and a second electrode is placed directly above the same eye. An optional third electrode or a sensor is placed in-between the first and second electrodes so that the three electrodes (or two electrodes and a sensor) substantially form a right angle. Collectively, to ease description, the term sensing unit refers to both electrodes and sensors. **FIGS. 2D-2G** depict additional embodiments of the wearable device, where the sensing units are arranged in various positions to sense eye movement of a single eye from a single side of a subject's face. In some embodiments, one of the sensing units is positioned at an outside corner of the eye (e.g., FIGS. 2C, 2E, 2G and 2H). In some embodiments, two sensing units are positioned above the eye (FIGS. 2D, 2F, 2G, 2H). In an embodiment, the wearable device is comprised of three electrodes or of two electrodes and a sensing unit, where a first electrode is placed to sense signal associated with vertical eye movement and a second electrode is placed to sense signal associated with horizontal eye movement. The sensing unit can be a sensor, such as a gyroscope, an accelerometer, an inertial mass unit or a magnetometer, or can be an electrode, which may be a common electrode or a reference electrode. In an embodiment, the electrode that senses signal associated with vertical eye movement is positioned substantially between the two other sensing units in the device. In an embodiment, the electrode for detection of signal associated with horizontal eye movement is positioned substantially between the two sensing units in the device or is one of the terminal or distal electrodes (e.g., it is not positioned substantially between the two other sensing units). As will be described below, signal from the three sensing units is collected and processed with an algorithm to determine vertical, horizontal and/or torsional eye movement.

The arrangement of sensing units depicted in **FIGS. 2A-2G** deviates from prior art devices, as depicted in **FIG. 2I**, where a pair of electrodes are aligned in a vertical plane v to detect signal associated with vertical eye movement, and a pair of electrodes are aligned in a horizontal plane *h* to detect signal associated with horizontal eye movement. The electrode pair in horizontal plane *h* are biocularly positioned. In contrast, the wearable device herein is configured for unilateral placement on the face for monocular placement of the sensing units. Since monocular corneo-retinal potential is lower than binocular corneo-retinal potential, the present device comprises circuitry to amplify the corneo-retinal potential signal and to improve the signal to noise ratio. The circuity permits use of a shared reference electrode, and permits the electrodes or sensors to not be perfectly aligned in the horizontal and vertical planes (h and v, respectively, in **FIG. 2I**). As will be described below, the electrodes or sensors, the circuitry and the algorithm operate to decouple the signals detected by the electrodes or sensors associated with horizontal and vertical eye movement from monocular corneo-retinal potential. In other embodiments, the electrodes or sensors detect electrical activity associated with extraocular or facial muscles associated with a single eye.

An embodiment of the circuitry of a device is shown in **FIG. 3A.** A device **300** is shown in schematic form, wherein the device is configured such that sensors, indicated by representative sensors **302**, **304,** and circuitry **306** are configured onto a single, wearable substrate **308** and are operably connected via wires **310.** Circuitry **306** comprises an analog front end **312** and a digital circuit **314.** Analog front end **312** comprises an amplifier **316** and a noise filtering circuit **318.** Digital circuit **314** comprises an analog to digital converter **320,** a microcontroller **322,** a digital signal processor **324** and, optionally, a transmitter **326,** which is a wireless transceiver.

As mentioned above, in some embodiments, the device may comprise a third sensor or a third electrode. In some cases, the third sensor is one configured to sense head movement, position and/or orientation of a subject. With reference to **FIG. 3B,** a third sensor **350** that may be additionally integrated into a wearable device is depicted In this embodiment, the third sensor is an accelerometer, a gyroscope, a magnetometer or an inertial mass unit. Panel **360** in **FIG. 3B** depicts exemplary head positions of a subject that may be detected by the third sensor. The panel shows how the inner ear position changes with movement of the head. The black dots, such as dots **362, 363,** show the otoconia within the semicircular canals, also known as the balance canals, and how they change position with head movement, position or orientation. Starting from the upper left in panel **360** and moving clockwise as indicated by the directional arrows, the top row depicts person moving to a head back position, then to a head up position, and then returning to a body up or upright position. By knowing the head position, direction and speed of acceleration that elicits nystagmus, the position of potential otoconia in the semicircular canals can be determined. With the addition of a third sensor, the wearable device may be configured to monitor both eye movement, including horizontal and/or vertical eye movement, and head movement, position and/or orientation of a subject. In an embodiment, the device is configured to monitor head movement, position and/or orientation along three axes. The circuitry of the device is operably coupled to the third sensor and it is configured to detect and/or discriminate head position, movement, and/or orientation based on signals received from the sensor.

In one embodiment, the third sensor also functions as a trigger to activate the one or more electrodes on the device to initiate detection of signal associated with eye movement. For example, a particular position, movement or orientation of the head detected by the third sensor signals the electrodes to begin recording electrical activity associated with eye movement. Alternatively, and as discussed below, the device or system can comprise a physical or electronic button to activate or deactivate the device.

As mentioned, a feature of the present device is its being configured to be applied to only one side of a subject's face, and to employ data obtained from sensors applied to only one side of a subject's face. What is meant by 'only one side of a subject's face' is that the device, when employed, is applied to only one side of one eye of a subject and is not applied to the side of each eye of a subject. The devices are configured to be operated with only first and second sensors, e.g., electrodes, applied to a single side of a subject's face, e.g., proximal to only a single eye. Additionally, in certain embodiments, the device may also include a third sensor configured to sense head movement, position and/or orientation of the subject, a photosensor configured to detect ambient light, a storage component, and/or a transmitter.

Before describing the software application with an algorithm to process signal detected by the device and to provide an interface for a user of the wearable device, the components of the device - *e.g.,* the substrate, the sensors and the circuitry, will be further described.

The constituent components of the wearable device may be integrated onto a single, unitary substrate. By substrate, it is meant an underlying material or layer that acts as a mechanical base on which the constituent components of the device may be mounted and fastened. The substrate may be a substantially flat surface where each constituent component occupies an area on the flat surface of the substrate. The substrate may be a substantially planar member, where each constituent component occupies an area on or within the planar substrate. In an embodiment, by "flat," it is meant that, for example, the substrate may be less than about 35 mm in thickness, and in other examples, it is less than about 30 mm, 28 mm, 27 mm, 26 mm, 25 mm, 24 mm, 23 mm, 22 mm, 21 mm or 20 mm in thickness. The constituent components of the device may be mounted, fastened, secured onto one or both of the flat sides of the substrate or may be embedded within the substrate.

The substrate may be comprised of any convenient material. For example, the substrate may be a printed circuit board. Alternatively, the substrate may be a flexible material. For example, the substrate may be a semi-rigid colloid such as silicone gel or hydrogel. Alternatively, the substrate may be cloth, such as canvas or duck cloth. Alternatively, the substrate may be a moldable plastic, such as a plastic cloth or the like. Alternatively, the substrate may be a laminate. Alternatively, the substrate may comprise a resin, such as a paper or a cloth combined with resin.

Constituent components of the device may be bonded, mounted, enclosed, and/or fastened onto or into the substrate in any convenient manner. For example, constituent components of the device may be bonded onto the substrate through electrochemical or chemical reactions. Alternatively, constituent components of the device may be secured, mounted and/or fastened onto the substrate using hardware fasteners. Hardware fasteners may include screws, rivets, bolts, pins, clamps, staples, PEM nuts, hook and loop fasteners (e.g., Velcro®) and the like. Alternatively, constituent components of the device may be secured, mounted and/or fastened onto the substrate by adhering them to the substrate. For example, constituent components of the device may be secured, mounted and/or fastened onto the substrate using glue, cement, paste and the like. Securing, mounting and/or fastening constituent components to the substrate using adhesives may in some cases include taping components to the substrate. Alternatively, constituent components of the device may be secured, mounted and/or fastened onto or into the substrate by sewing them onto or into the substrate. When the substrate is a printed circuit board, constituent components of the device may, for example, be soldered onto the printed circuit board.

The substrate may take any convenient shape. For example, the substrate may be substantially ovoid shaped, or substantially teardrop shaped, or substantially crescent shaped. Alternatively, the substrate may form two "arms" (e.g., rectangular arms, curvilinear arms, etc.) oriented at approximately right angles from each other. That is, the substrate may take a substantially "L" shape, where the edges of a such a shape may be linear or curvilinear, as desired. The size of the substrate may be measured by the surface area of one of the "flat" sides of the substrate. The area of the substrate may vary, and in some instances may range from about 2-70 cm$^2$, about 4-65 cm$^2$, about10.0 to 65.0 cm$^2$, about 10-50 cm$^2$, about 15-40 cm$^2$, or about 20.0 cm$^2$. The length and width of the substrate may vary, and in some instances ranges from about 0.1-10 cm, about 0.2-8 cm, about 1-8 cm, about 3 to 8 cm, or about 5.0 cm. When the substrate is configured in a substantially "L" shape, such that its shape comprises two "arms" oriented at approximately right angles from each other, in some instances, each of the "arms" may have a length of between about 1-15 cm, 2-10 cm, or 3-8 cm and a width of 0.5 to 4.0 cm. When the substrate is configured in such an "L" shape, the length of each of the "arms" of the substrate may be the same length or the lengths may differ. For example, the lengths of the two arms of the substrate may differ by between about 0.1 to 4.0 cm, about 0.5-3 cm, 0.5-2 cm or 1-2 cm. An exemplary L shaped device has a first arm with a length of between about 50-65 cm, 52-60 cm, or 55-60 cm, and a second arm that has a length that is about 5%, 8%, 10%, 12%, 15% or 20% shorter that the length of the first arm.

In certain embodiments, the shape of the substrate is substantially determined based on the desired positions of the first and second sensors mounted onto the substrate. That is, the substrate is shaped such that when the device is worn by a human subject, the first and second sensors are positioned proximal to one eye of the subject. In certain embodiments, to sense movement of one of the subject's eyes, the substrate may be configured such that the first and second sensors may be positioned proximal to one of the subject's eyes. In particular, they may be positioned proximal to the eye, asymmetrically with respect to a hypothetical vertical axis intersecting the pupil of the proximal eye when looking straight ahead, and asymmetrically with respect to a hypothetical horizontal axis intersecting the pupil of the proximal eye also when looking straight ahead. In certain embodiments, the positions in which the first and second sensors are mounted onto or into the substrate are determined such that the distance between the first and second sensors is substantially maximized. That is, in certain embodiments, the longest dimension of the substrate is substantially determined by the desired distance between the first and second sensors. In some instances, the shape of the substrate with first and second sensors mounted thereon is configured such that when the device is worn by human subjects, one sensor is mounted substantially above or substantially below one eye of the subject and the other sensor is mounted substantially to the side of the same eye of the subject. In a given configuration, the distance between the first and second sensors may vary, ranging in some instances from about 0.25-8 cm, about 0.5-8 cm, or about 0.50 cm to 5.60 cm, or about 4.0 cm.

The wearable device also comprises first and second sensors, which are configured to sense movement of one of the subject's eyes. The first and second sensors may sense movement of one of the subject's eyes by measuring electrical activity associated with eye movement. In certain embodiments, the first and second sensors may sense the movement of the subject's eye by measuring the difference in electrical potential between the cornea and the retina of the subject's eye. In other embodiments, the first and second sensors may sense the movement of the subject's eye by measuring the electrical activity of the subject's muscles, for example, the extraocular and facial muscles. The first sensor comprises one or more electrodes, and the second sensor comprises one or more electrodes. In certain embodiments, the first sensor comprises a single first electrode and the second sensor comprises a single second electrode. Alternatively, in some embodiments, the first sensor comprises two or more first electrodes, and the second sensor comprises two or more second electrodes. In particular, the one or more first electrodes and the one or more second electrodes may be surface electrodes, in which case they may be dry electrodes. In another embodiment, the one or more firsts electrodes and the one or more second electrodes may be wet electrodes. In certain embodiments, the device may be configured to monitor the subject's eye movement based on signals from the first and second sensors continuously and/or in near real time.

When the first and second sensors comprise one or more first electrodes and one or more second electrodes, respectively, the electrodes may be any convenient electrode. By electrode, it is meant an electrical conductor used to make contact with a nonmetallic substance. In some instances, the electrodes are integrated into the device such that one end of the electrode is in electrical contact with the subject, and the other end of the electrode is electrically connected to the circuitry of the device. In some instances, the electrodes have a proximal and distal end, wherein the proximal end is electrically connected to the circuitry component of the device, and the distal end is in electrical contact with the subject when in use. Thus, in the present invention, an electrode may be used to conduct electrical signals generated by the subject and sensed by the electrode to the circuitry component of the device. In certain embodiments, the one or more first electrodes and the one or more second electrodes may measure the difference in electrical potential between the cornea and the retina of the subject's eye. In other embodiments, as discussed above, the one or more first electrodes and the one or more second electrodes may measure the electrical activity of the subject's muscles, for example, the extraocular and facial muscles.

In certain embodiments, the one or more first electrodes and the one or more second electrodes are surface electrodes. By surface electrodes, it is meant electrodes that they are applied to the outer surface of a subject to measure electrical activity of tissue proximal to the electrode. That is, surface electrodes are electrodes that do not penetrate the skin of a subject. Surface electrodes may be any convenient commercially available surface electrode, such as the Red Dot™ line of electrodes that are commercially available from 3M™ or Disposable Surface Electrodes that are commercially available from Covidien or ECG Pre-Gelled electrodes that are commercially available from Comepa. In certain embodiments, surface electrodes may be comprised of a backing material that is a cloth, a foam, a plastic tape, a plastic film, or any other convenient material. Surface electrodes may be applied to the surface of the subject using an adhesive. The strength of the adhesive may vary as desired. The adhesive may itself be conductive. The surface electrodes may be designed to be conveniently repositioned on a subject as needed to improve functioning of the device.

In certain embodiments, the surface electrodes may be dry electrodes. By dry electrode, it is meant that the electrodes do not require the application of any gel or other fluid between the subject's skin and the distal surface of the electrode for the electrode to function in the device. In certain embodiments, the dry surface electrodes do not require any skin preparation, such as skin abrasion, in order to function when applied to the surface of a subject. When the electrodes are not dry electrodes, gel or other similar fluid is be applied to the surface of the subject between the skin and the electrode in order to promote electrical connectivity between the subject and the surface electrode. In certain instances, dry electrodes promote long term use of the electrodes and therefore long-term use of the device by alleviating the need to reapply gel or similar fluid as the gel or other fluid dries. Wet electrodes offer an advantage of stable signal for longer periods of time.

The surface electrodes may take any convenient shape. In certain embodiments, the surface electrodes may be substantially round or substantially rectangular or substantially ovoid, or substantially teardrop shaped. The surface electrodes may cover an area of the surface of a subject that varies, where in some instances the covered area ranges from 0.05 to 10.0 $cm^2$, such as 1.0 $cm^2$. The shape or the surface area of the first electrode may differ from the shape or the surface area of the second electrode. For example, the first electrode may be substantially circular, and the second electrode may be substantially rectangular. The first electrode may be larger or smaller than the second electrode, where in some instances the magnitude of any difference ranges from about 0-12 $cm^2$, about 0-9.95 $cm^2$, about 0-7 $cm^2$, about 0 to 3.75 $cm^2$, or about 2.0 $cm^2$.

As discussed above, in certain embodiments, the shape of the substrate is substantially determined based on the desired positions of the first and second sensors mounted onto the substrate. When the first and second sensors are configured to be first and second electrodes, the positions of the first and second electrodes mounted on the substrate determine where on the subject the first and second electrodes are located. The location of the first and second electrodes on the subject determine, in part, the electrical signals associated with eye movement of the subject that can be sensed by the first and second electrodes. The positions of the first and second electrodes mounted on the substrate can be described based on the distance between the center of the first electrode and the center of the second electrode and in some instances ranges from about 0.25-8 cm, about 0.5-8 cm, or about 0.1-7.0 cm, or about 4.0 cm.

When the substrate is configured in substantially an "L" shape, such that its shape comprises two "arms" oriented at approximately right angles from each other, one sensor may be mounted on each of the "arms." In certain instances, the sensors are mounted on each "arm" near the furthest point away from the vertex of the "L" shape. The sensors are mounted on the substrate such that when the device is worn by a subject, one of the two sensors is located substantially above one eye of the subject and the other sensor is located substantially to the side of the same eye of the subject. In such embodiments, the sensors are positioned asymmetrically with respect to a hypothetical vertical axis intersecting the pupil of the eye when looking ahead, and asymmetrically with respect to a hypothetical horizontal axis intersecting the pupil of the eye when looking ahead.

As mentioned, the devices are wearable on the face of a subject. For example, a device can be worn by a subject outside of a clinical setting, such as at home or at work. In an embodiment, the device comprises a single wearable patch or in other embodiments, the device comprises more than one wearable patch. By comprising a single wearable patch, it is meant that the substrate on which the device is mounted is itself integrated into a single wearable patch. By comprising more than one wearable patches, it is meant that the components that comprise the device are integrated onto more than one wearable patch, such that when worn, each wearable patch is physically separated from each other and may be electrically connected via wires or may be in wireless communication with each other.

The single wearable patch may take any convenient form and may include any convenient material or materials. For example, the single wearable patch may include a flexible material, such as a cloth-based material, that can be shaped over parts of the face of the subject. The single wearable patch can also be formed from layers of the same or different materials or may be a laminate of the same or different materials. When the device is configured to comprise a single wearable patch, the wearable patch may be adhered to a facial location of the subject or may be attached to a facial location of the subject using non-adhesive material. In certain embodiments, the single wearable patch may be adhered to a facial location of the subject using a glue or a cement or a paste. In certain embodiments, the single wearable patch may be attached to a facial location of the subject not by using an adhesive but instead by using, for example, a tensioning method, such as an elastic band or an eye glass frame. In some embodiments, the single wearable patch may be flexible so as to be fitted to a facial location of the subject. By flexible so as to be fitted to a facial location, it is mean that the surface of the single wearable patch is not rigid or firm but instead may be positioned so as to follow the pattern of and be aligned with the non-flat contours of a subject's face. In some embodiments, the single wearable patch may be moldable so as to be form-fitted to a facial location of the subject. By moldable so as to be form-fitted to a facial location of the subject, it is meant that the surface of the single wearable patch can be manipulated and formed so as to follow the pattern of and be aligned with the non-flat contours of a subject's face and, further, when so formed, will retain such molded position. In some embodiments, the single wearable patch may be configured to be torn so as to be fitted to a facial location of the subject. By being configured to be torn so as to be fitted to a facial location of the subject, it is meant that the material of the wearable patch is configured so as to guide a user to tear a flat surface of the wearable patch such that the otherwise flat surface of the wearable patch may better accommodate being applied to a more substantially rounded or curved surface of a subject's face.

An exemplary embodiment of a single wearable patch or single wearable device is illustrated in **FIG. 4**. Device **400** comprises a first sensor and a second sensor, **402**, **404**, respectively, and circuitry **406**. Circuitry **406** comprises an electronic component **408** and wires or traces **410,** all shown in phantom because they are embedded in the single unitary substrate **412**. In an embodiment, the unitary substrate is a flexible polymer, such as silicone rubber, and the components are embedded within the flexible polymer. Electronic component **408** can be, for example, a chip or printed circuit board comprising one or more of the electronic components described herein. The first and second sensors in this embodiment are shown to each be a single electrode, each with a snap type fastener, such as fastener **414** on sensor **404**. Fastener **414** removably engages with a member on the skin contact surface of the device, such as member **416**. Device **400** is affixed to the skin of a subject by the electrodes that are secured, such as by an adhesive or by tape, to the skin, and by the fastening member on the device that removably engages with a mating member on an electrode. Device **400** may comprise an adhesive portion on the skin contact side in the region of the electronic component, if there is no adhesive-backed third sensor electrode in this region of the device. Device **400** may also comprise fiducials or alignment markings to guide positioning of the device on the face of a user. For example, the alignment markings might indicate alignment with the pupil. The alignment marking(s) may take the form of a removable layer that is peeled from the external surface of the device after it is affixed to the face.

From the embodiment in **FIG. 4,** a variety of device configuration can be appreciated. In one embodiment, the wearable device comprises a reusable, non-disposable unitary substrate comprising the circuitry. First and second sensors or electrodes are removably attachable to the substrate, and may be disposed after use. The reusable, unitary substrate is attached to a fresh pair of unused electrodes or sensors for each subsequent use. In another embodiment, the substrate and electrodes of the device are disposable, and a majority of the circuitry is removable from the substrate for reuse. An example of this embodiment is depicted in FIG. 1B, where an electronic component of the circuitry is removable from the substrate. Devices that are entirely disposable or entirely reusable are also contemplated. In another configuration, the wearable device is comprised of a first sensor and a second sensor and an electronic component, where the electronic component is on a substrate separate from the substrate(s) on which the first sensor and second sensor are affixed. Circuitry between the separate substrates and sensor operably connects the sensor with the electronic component. Also contemplated is a device as described and comprising a skin adhesive layer and additional materials to assemble separate components together to form the device and affix it to the skin.

Accordingly, in embodiments, a device for adhesion to skin of a subject is provided. The device comprises a unitary substrate with a first arm and a second arm that join at a connection point, a first electrode positioned on the first arm and a second electrode positioned on the second arm. The device also comprises an electronic component, such as a data collection circuit, removably affixed at the approximately the connection point. The first and second electrodes are integral with a bottom surface of each of the first arm and the second arm, respectively, and electrically connected to the electronic component when affixed to the substrate. The device may also comprise an adhesive on a bottom surface of each arm for contact with skin, and a flexible overlay covering the external, outward surfaces of the electrodes and the electronic component. The arms of the device may be flexible or rigid, depending on the materials from which the substrate is formed. In another embodiments, a device for adhesion to skin of a subject comprises a unitary substrate, which can be a layered substrate, with a first arm and a second arm and a data collection member or electronic component. First and second electrodes are removably affixed at separate individual connection points to the substrate, where the connection points are on a skin contact surface of each of the first arm and the second arm. The electronic component inserted onto or into the substrate is in electrical connection with the first and second electrodes. An adhesive may be present on the skin contact surface of the electrodes, and a flexible overlay material may cover the external, outward surfaces of the electrodes and the electronic component.

As described above, the first sensor may comprise two or more electrodes, and the second sensor may comprise two or more electrodes. In some instances, the first and second electrodes comprise two or more groups of first and second sensors. In such instances, a group of first and second sensors may each comprise a pair of electrodes. In some instances, a group of first and second sensors may each comprise more than two electrodes. In some cases, each group of first and second electrodes comprises the same number of electrodes, and in some cases, each group of first and second electrodes comprises a different number of electrodes.

When an embodiment of the device comprises groups of first and second sensors, the groups of first and second sensors may be geometrically arranged in different configurations. In some instances, groups of first and second sensors may be geometrically arranged symmetrically with respect to a vertical or horizontal axis through the pupil of the subject, and in some instances, groups of first and second sensors may be geometrically arranged asymmetrically with respect to a vertical or horizontal axis through the pupil of the subject. In some instances, one or more groups of first and second sensors may be geometrically arranged to isolate vertical eye movements of the subject. In some instances, one or more groups of first and second sensors may be geometrically arranged to isolate horizontal eye movements of the subject. In some instances, one or more groups of first and second sensors may be geometrically arranged to isolate torsional eye movements of the subject. In some instances, multiple groups of first and second sensors may be geometrically arranged to isolate vertical, horizontal and torsional eye movements of the subject.

When an embodiment of the device comprises groups of first and second sensors, signals from the different groups of first and second sensors may be received by the circuitry simultaneously. That is, the circuitry may receive all of the signals detected by each group of first and second sensors. In other instances, signals from the different groups of first and second sensors may be received by the circuitry in a time-gated manner. That is, in some instances, signals from the different groups of first and second sensors may be multiplexed prior to being received by the circuitry. In some instances, signals from the different groups of first and second sensors may be multiplexed such that the circuitry receives signals from each group of sensors in substantially equal durations of time. In other instances, signals from the different groups of first and second sensors may be multiplexed such that the circuitry receives signals from one group of sensors for a greater duration of time than the circuitry receives signals from another group of sensors.

Turning now to the circuity of the device, it is preferred that the circuitry be operably coupled to the first and second sensors and be configured to detect horizontal and/or vertical eye movements based on signals from the first and second sensors. The circuitry may comprise an analog front end and a digital circuit. The analog front end receives signals from the sensors and may include a noise filtering circuit and an amplifier circuit. The digital circuit may include an analog to digital converter circuit configured to convert the analog signal output from the analog front end into a digital signal as well as a microcontroller. In some embodiments, the digital circuit may also include a digital signal processor to further process the signal output from the analog front end.

In embodiments of the device that comprise a third sensor, the circuitry is operably coupled to the third sensor and is further configured to detect head movement, position and/or orientation based on signals from the third sensor. In other embodiments, the device further comprises a photosensor, and the circuitry is operably coupled to the photosensor and is configured to detect ambient light based on signals from the photosensor. In other embodiments, the device further comprises a storage component, and the circuitry is operably coupled to the storage component; and the circuitry and the storage component are configured to record eye movement data and head movement, position and/or orientation data onto the storage component. In other embodiments, the device further comprises a transmitter, and the circuitry is operably coupled to the transmitter; and the circuitry and the transmitter are configured to transmit eye movement data and head movement, position and/or orientation data.

By circuitry, it is meant an electronic circuit, in which electrical signals are conveyed via electrical conductors and the voltage and/or current of the electrical signals may be manipulated by electronic components, such as, for example, resistors or capacitors or voltage sources or current sources and the like. The circuitry may be further comprised of electrical components that are semiconductor devices, such as transistors or integrated circuits and the like. The electronic components comprising the circuitry may consist of both analog and digital electronic components.

In embodiments, analog components of the circuitry may comprise one or more amplifiers, such as, for example, operational amplifiers, and one or more analog filters, such as, for example, low-pass filters, high-pass filters or band-pass filters. In embodiments, one or more amplifiers is used in the circuit to amplify electronic signals originating from first and second electrodes. In particular, one or more amplifiers and analog filters may be used in the circuit to amplify and filter aspects of the signals received from first and second electrodes that are associated with horizontal or vertical eye movement. In some embodiments, one or more amplifiers and analog filters may be used in the circuit to amplify and filter aspects of the signals received from first and second electrodes that are associated with torsional eye movements or characteristic eye movements, such as for example, neutral gaze, leftward gaze, rightward gaze, upward gaze and downward gaze, or, in other embodiments, eye movements associated with a nystagmus event. In some embodiments, one or more amplifiers and analog filters may be used in the circuit to amplify and filter aspects of the signals received from first and second electrodes that facilitate distinguishing between characteristic eye movements of horizontal nystagmus events, vertical nystagmus events and torsional nystagmus events. In some embodiments, one or more amplifiers and analog filters may be used in the circuit to amplify aspects of the signals received from first and second electrodes that are associated with characteristic eye movements of nystagmus events associated with benign paroxysmal positioning vertigo, or characteristic eye movements of nystagmus events associated with Menière's disease, or characteristic eye movements of nystagmus events associated with vestibular neuritis. In addition, one or more amplifiers and analog filters may be used in the circuit to condition the signals received from the first and second electrodes such that they can be further processed by additional electronic components of the circuit.

In some embodiments, digital components of the circuitry may include an analog to digital converter, a microcontroller and a digital signal processor. By analog to digital converter, it is meant an electronic circuit used to convert electrical signals from an analog format or encoding to a digital format or encoding. In certain embodiments, an analog to digital converter may be used to convert analog signals that have already been processed by the analog electronic components of the circuit into digital signals, such that the resulting digital signals can be further processed by additional electronic components of the circuit. By microcontroller, it is meant an electronic circuit that comprises one or more processors, memory and one or more input/output interfaces. In certain embodiments, the microcontroller may be programmed to further process the digital signal corresponding to the signal measured by the first and second electrodes as well as the digital signal corresponding to the signal measured by the third sensor, such as an accelerometer, and the digital signal corresponding to the signal measured by the photosensor. The microcontroller may also be programmed to facilitate transmitting a digital signal via a transmitter or to facilitate storing a digital signal onto an external storage component. The microcontroller may also be programmed to fetch ADC converted data, to schedule data communication, and to facilitate local signal processing. By digital signal processor, it is meant a special purpose microprocessor that is optimized for performing signal processing operations, such as measuring digital signals, comparing digital signals against reference waveforms, filtering digital signals, or compressing digital signals and the like. In certain embodiments, the digital signal processor may be used to: scale and bias raw sensor measurements; identify characteristic waveforms by comparing measured waveforms against a reference waveform; identify specific characteristics of measured waveforms; or compress the digital representation of the waveform prior to transmitting or storing the waveform. For example, when the digital signal processor is used to identify characteristic waveforms of the digital signal corresponding to the signal measured by the first and second sensors, the digital signal processor may compare the measured signal against reference waveforms corresponding to the eye movements associated with horizontal or vertical eye movement, or reference waveforms corresponding to eye movements associated torsional eye movements, or reference waveforms corresponding to characteristic eye movements, such as for example, neutral gaze, leftward gaze, rightward gaze, upward gaze and downward gaze, or reference waveforms corresponding to characteristic eye movements associated with a nystagmus event, or reference waveforms corresponding to characteristic eye movements associated with horizontal nystagmus events, vertical nystagmus events and torsional nystagmus events, or reference waveforms corresponding to characteristic eye movements of nystagmus events associated with benign paroxysmal positioning vertigo, or reference waveforms corresponding to characteristic eye movements of nystagmus events associated with Menière's disease, or reference waveforms corresponding to characteristic eye movements of nystagmus events associated with vestibular neuritis.

In some embodiments, the electronic components described above may be provided in the form of integrated circuits. Alternatively, in some embodiments, one or more of the electronic components described above may be provided in the form of a configurable integrated circuit. For example, one or more of the electronic components described above may be provided on a field programmable gate array that has been configured to implement identical functionality.

In some embodiments, the electronic components that make up the circuitry are commercially available, "off-the-shelf" components. For example, integrated circuits comprising operational amplifiers, analog filters, analog to digital converters, microcontrollers and digital signal processors are commercially available from Texas Instruments or Analog Devices and Marvell. Field programmable gate arrays that can be configured to implement one or more of the electronic components described above are commercially available from Xilinx or Intel and Altera.

The algorithm that processes signal from the circuitry is able to detect horizontal and/or vertical eye movements based on signals from the first and second sensors. In some embodiments, the algorithm may be configured to detect additional eye movements based on signals from the first and second sensors, such as torsional eye movements, one or more specific patterns of eye movement sensor data comprising one or more characteristic eye movements, including, for example, recognizing neutral gaze, leftward gaze, rightward gaze, upward gaze and downward gaze, or, a nystagmus event, including horizontal nystagmus events, vertical nystagmus events and torsional nystagmus events, or nystagmus events associated with benign paroxysmal positioning vertigo, or nystagmus events associated with Menière's disease, or nystagmus events associated with vestibular neuritis. In certain embodiments, when the first and second sensors are positioned proximal to one eye of a human subject, the algorithm may be configured to detect eye movements by first receiving electrical signals from the first and second sensors in an analog format that is correlated with eye movement. By a signal correlated with eye movement, it is meant, for example, a signal that represents the difference in electrical potential between a cornea and a retina of the subject or an electrical signal that represents the electrical activity of muscles. By electrical activity of muscles, it is meant that the first and second sensors are positions and configured so as to measure the electrical activity of extraocular and facial muscles associated with eye movement. Upon receiving such analog electrical signals from the first and second sensors, the circuitry may be configured to selectively amplify the signal using an analog amplifier, as described above, to particularly amplify, for example, the part of the signal that comprises the difference in electrical potential between a cornea and a retina of the subject or the part of the signal that comprises the electrical activity of extraocular and facial muscles associated with eye movement. In one embodiment, signal that comprises the electrical activity of extraocular and facial muscles is removed or subtracted from the signal collected from the device.

Upon amplifying the signal, the circuitry may be configured to filter the amplified signal using an analog filter, as described above, to exclude and remove parts of the analog electronic signal that do not correspond to, for example, measurements of the difference in electrical potential between a cornea and a retina of the subject or measurements of electrical activity of muscles such as the electrical activity of extraocular and facial muscles associated with eye movement. Upon amplifying and filtering the analog electronic signal, the circuitry may be configured to convert the analog signal into a digital signal using an analog to digital converter, as described above. Upon converting the signal into a digital signal, the circuitry may be configured to measure aspects of the signal using a digital signal processor, as described above, to identify characteristics of the digital signal that are associated with horizontal or vertical eye movements. In certain embodiments, the digital signal processor may be configured to identify additional characteristics of the digital signal that are associated with eye movements, such as torsional eye movements, one or more specific patterns of eye movement sensor data comprising one or more characteristic eye movements, including, for example, recognizing neutral gaze, leftward gaze, rightward gaze, upward gaze and downward gaze, or, a nystagmus event, including horizontal nystagmus events, vertical nystagmus events and torsional nystagmus events, or nystagmus events associated with benign paroxysmal positioning vertigo, or nystagmus events associated with Menière's disease, or nystagmus events associated with vestibular neuritis.

As mentioned above, the device may include a third sensor configured to sense head movement, position and/or orientation of the subject. By sensing head movement, position and/or orientation of the subject, it is meant that the device is configured to detect when the head of a subject is translated back and forth, up or down or side to side in space, as well as when the head is rotated from side to side or back and forth or up and down, or combinations thereof.

In some embodiments, the third sensor may be an accelerometer. By accelerometer, it is meant a component that measures the acceleration of a body, such as acceleration on three dimensions in space. The accelerometer may comprise a mechanical-electronic device or a microelectromechanical system (MEMS). For example, an accelerometer may utilize the piezoelectric effect to measure acceleration. Typically, the accelerometers integrated into the device are commercially available, "off-the-shelf" components. For example, integrated circuits comprising accelerometers are commercially available from Analog Devices or Texas Instruments or Marvell.

Alternatively, the third sensor may be a gyroscope. By gyroscope, it is meant a component that measures the changes in the position or rotation of a body, such as changes in the orientation of the body in three dimensions in space. The gyroscope may comprise a mechanical-electronic device or a microelectromechanical system (MEMS). For example, a gyroscope may be a vibrating structure gyroscope designed to utilize the piezoelectric effect to react to Coriolis force and thereby measure rotation of the sensor. Typically, the gyroscopes integrated into the device are commercially available, "off-the-shelf" components. For example, integrated circuits comprising gyroscopes are commercially available from Analog Devices or Texas Instruments or Marvell. The third sensor can also be a magnetometer or an inertial mass unit.

In some embodiments, the device may be configured to monitor the subject's head movement, position and/or orientation based on signals from the third sensor continuously and/or in near real time. By monitoring the subject's head movement, position and/or orientation continuously, it is meant that the device may be configured to monitor the subject's head movement, position and/or orientation based on substantially every signal sensed by the third sensor. By monitoring the subject's head movement, position and/or orientation in near real time, it is meant that the device is configured to analyze and evaluate the subject's head movement, position and/or orientation nearly in real time after the signals are sensed by the third sensor and received by the circuitry.

Certain embodiments of the device may include a photosensor configured to sense ambient light in the vicinity of the subject. By ambient light in the vicinity of the subject, it is meant the light intensity of light that is proximal to the subject wearing a device configured to include a photosensor. In some cases, ambient light also includes other characteristics of light, such as changes in light intensity or changes in wavelength characteristics of light proximal to the subject.

By photosensor, it is meant an electronic component capable of converting light into electronic current, such as a photodiode, such that the resulting electronic current can be measured by the circuitry of the device.

Typically, the photosensors integrated into the device are commercially available, "off-the-shelf" components. For example, integrated circuits comprising photosensors are commercially available from Texas Instruments or Analog Devices or Marvell.

In some embodiments, the device may be configured to monitor ambient light in the vicinity of the subject based on signals from the photosensor substantially in real time. By monitoring the ambient light in the vicinity of the subject in near real time, it is meant that the device is configured to analyze and evaluate characteristics of ambient light in the vicinity of the subject nearly in real time after signals are sensed by the photosensor and received by the device.

The device may also comprise a storage component. In this embodiment, the circuitry and the storage component are configured to record eye movement data and head movement, position and/or orientation data onto the storage component. By record eye movement data and head movement, position and/or orientation data onto the storage component, it is meant electronically retain signals sensed by the first and second sensors and the third sensor or processed signals or information derived from signals sensed by the first and second sensors and the third sensor onto a persistent memory storage device such that the stored data can be accessed at a later time.

By storage component, it is meant an electronic component capable or having electronic data written onto it and read from it, such that data written thereon persists over time in a manner that it can be accessed and read at a later time. Typically, the storage components integrated into the device are commercially available, "off-the-shelf" components. For example, flash memory storage components are commercially available from Intel or Samsung or Toshiba.

The storage component may be removable. For example, the storage component may be a removable memory card such as an SD card or the like. By removeable, it is meant that the storage component may be configured such that it can be physically and electronically separated and removed from the circuitry of the device and later physically and electronically reintegrated into the device. The storage component might be separated from the device so that data on the storage device can be read and downloaded onto a remote computer system.

In certain embodiments, the device may be configured to record eye movement data and head movement, position and/or orientation data onto the storage component in near real time. By recording eye movement data and head movement, position and/or orientation data onto the storage component in near real time, it is meant that the device is configured to store signals sensed by the first and second sensors and the third sensor or signals or data derived from the signals sensed by the first and second sensors and the third sensor in nearly in real time after signals are sensed by the first and second sensors and third sensor and received by the circuitry.

The device may also comprise a transmitter. In this embodiment, the circuitry and the transmitter are configured to transmit eye movement data and head movement, position and/or orientation data that originated from the first and second sensors and the third sensor, respectively. By transmitter, it is meant an electronic component that receives an electronic signal as input and conveys such signal to a remote receiver.

In some embodiments, the transmitter may be a wireless transmitter. By wireless transmitter, it is meant that the transmitter receives an electronic signal and produces electromagnetic waves via an antenna corresponding to that signal that can be received by a receiver that is remote, meaning electronically and physically separated from the device. In some instances, the wireless transmitter may be a wireless network interface controller, meaning, for example, a device capable of connecting via radio waves to a radio-based computer network. Alternatively, the wireless transmitter may be a Bluetooth interface controller, meaning, for example, a device capable of connecting via the radio waves to a Bluetooth-enable remote device. Typically, the transmitters integrated into the device are commercially available, "off-the-shelf" components. For example, wireless network interface controllers are commercially available from Intel or Nordic or Qualcomm, and Bluetooth interface controllers are commercially available from Motorola or Nordic or Qualcomm.

In some embodiments, the device may be configured to transmit eye movement data and head movement, position and/or orientation data via the transmitter in near real time. By transmitting eye movement data and head movement, position and/or orientation data via the transmitter in near real time, it is meant that the device is configured to transmit signals sensed by the first and second sensors and the third sensor or signals or data derived from the signals sensed by the first and second sensors and the third sensor in nearly in real time after signals are sensed by the first and second sensors and third sensor and received by the circuitry.

In an alternative embodiment of the device, the device comprises first and second sensors configured to sense eye movement of a subject; and a transmitter configured to transmit signals sensed by the first and second sensors to remote circuitry configured to receive signals transmitted by the transmitter and to detect horizontal and vertical eye movement based on signals from the first and second sensors. By first and second sensors configured to sense eye movement of a subject, it is meant first and second sensors as described in detail above. By transmitter configured to transmit signals sensed by the first and second sensors, it is meant a transmitter as described in detail above. By remote circuitry, it is meant any convenient circuitry that may be configured to receive and process signals measured from the first and second sensors. By remote, it is meant a location apart from the components that are interacting with the subject during use. For example, a remote location could be another location, e.g., different part of a room, different room, etc., in the same vicinity of the subject, another location in a vicinity different from the subject, e.g., separate portion of the same building, etc., another location in a different city, state, another location in a different country, etc. As such, when one item is indicated as being "remote" from another, what is meant is that the two items are at least in different locations, not together, e.g., are one to five or more feet apart, such as ten or more feet apart, including 25 or more feet apart.

Unlike the embodiments of the devices described above, this alternative embodiment of the device may not comprise circuitry. Instead, as described above, the device comprises a transmitter that transmits signals to remote circuitry configured to receive signals from the transmitter and detect horizontal and vertical eye movements based on signals received from the transmitter. In some embodiments, the remote circuitry may be configured to detect other eye movements, such as torsional eye movements, one or more specific patterns of eye movement sensor data comprising one or more characteristic eye movements, including, for example, detecting neutral gaze, leftward gaze, rightward gaze, upward gaze and downward gaze, or, a nystagmus event, including horizontal nystagmus events, vertical nystagmus events and torsional nystagmus events, or nystagmus events associated with benign paroxysmal positioning vertigo, or nystagmus events associated with Menière's disease, or nystagmus events associated with vestibular neuritis.

In some embodiments, the alternative embodiment further comprises a third sensor configured to sense head movement, position and/or orientation of the subject. By third sensor, it is meant the third sensor as described in detail above. In such an embodiment, the transmitter is further configured to transmit signals sensed by the third sensor to remote circuitry configured to receive signals from the transmitter and detect head movement, position and/or orientation by the subject based on signals received from the transmitter.

In some embodiments, the alternative embodiment further comprises a photosensor configured to sense ambient light in the vicinity of the subject. By photosensor, it is meant the photosensor as described in detail above. In such an embodiment, the transmitter is further configured to transmit signals sensed by the photosensor to remote circuitry configured to receive signals from the photosensor and detect ambient light in the vicinity of the subject based on signals received from the transmitter.

Where desired, the devices described herein may include ay one of a variety of different types of power sources that provide operating power to the device components, e.g., as described above, in some manner. The nature of the power source may vary and may or may not include power management circuitry. In some instances, the power source may include a battery. When present, the battery may be a onetime use battery or a rechargeable battery. For rechargeable batteries, the battery may be recharged using any convenient protocol, including, but not limited to, wireless charging protocols such as inductive charging. In some applications, the device may have a battery life ranging from 0.1 hours to 120 days, from 14-30 days, from 8 hours to 30 days, from 8 hours to 12 days, from 12 hours to 24 hours, from 0.5 to 10 hours.

As described in detail above, in some embodiments, the first and second sensors, the third sensor and the circuitry are all integrated onto a single substrate, such as, as described above, a printed circuit board. Further, in other embodiments, the single printed circuit board may further comprise a storage component or a wireless network interface controller or a photosensor, each of which may be integrated onto the printed circuit board.

Additionally, in some embodiments, the device may be waterproof. By waterproof, it is meant that the device is substantially resistant to water. That is, the device will continue to function correctly and consistently notwithstanding the presence of water proximal or on the device. For example, the device may be configured to function when worn by a subject outside in the rain or in a high humidity environment. In certain embodiments, the device may be configured to be waterproof by encasing the device in a housing, such as a plastic housing that itself is substantially waterproof.

In another aspect, a system for detecting eye movement of a subject is provided. The system comprises a wearable device, such as those described above, and a software application. In an embodiment, the software application is downloadable to a mobile device. The software application comprises one or more of (i) an algorithm for analysis of data from the wearable device; (ii) connectivity to a data storage; (iii) a user interface comprising an option to transmit data to a caregiver; and (iv) ability, using virtual reality, of administering a battery of classical nystagmus tests remotely. A particular embodiment of the system is comprised of a wearable device comprising first and second sensors configured to sense eye movement of a subject and circuitry operably coupled to the sensors and configured to detect horizontal and vertical eye movements based on signals from the sensors; and a software application installable onto or installed on a mobile device comprising a processor operably coupled to a memory that includes instructions stored thereon for interfacing with the wearable device. In an embodiment, the software application comprises an algorithm for analysis of signal obtained by the wearable device and transmitted to the mobile device. An exemplary algorithm is described below. The system may also comprise a storage component. The wearable device and the mobile device are operably coupled such that data originating from the first and second sensors are accessible by or on the mobile device. Additionally, in certain embodiments, the system may also include a display. Additionally, in certain embodiments, the wearable device component of the system may also include a third sensor configured to sense head movement, position and/or orientation of the subject; and a photosensor configured to detect ambient light.

In an alternative embodiment of systems for detecting eye movements of a subject, the system comprises a wearable device comprising first and second sensors configured to sense eye movement of a subject and a transmitter configured to transmit signals sensed by the first and second sensors to remote circuitry configured to receive signals transmitted by the transmitter and to detect horizontal and vertical eye movement based on signals from the first and second sensors, and a software application installable on a mobile device that comprises a processor operably coupled to a memory that includes instructions stored thereon for interfacing with the wearable device as well as an additional storage component, wherein the wearable device and the mobile device are operably coupled such that data originating from the first and second sensors are accessible to the mobile device. In some embodiments, the wearable device in the system may also include a third sensor configured to sense head movement, position and/or orientation of the subject; and a photosensor configured to detect ambient light.

The first and second sensors, the third sensor and the photosensor are each described above. The mobile device, additional storage and display components are now briefly described. The mobile device is operably coupled with the wearable device such that data originating from the first and second sensors are accessible to the mobile device. For example, the mobile device and the wearable device may be operably coupled via a wired or wireless connection. By "mobile" is meant that the mobile device can be moved by the subject during use. For example, a mobile device could be carried in the subject's hand or the subject's pocket while the system is in use. Alternatively, the mobile device could be held by someone other than the subject during use, such as a health care provider. The mobile device includes a processor that is operably coupled to a memory that includes instructions stored thereon for interfacing with the device as well as an additional storage component. By operable coupling between the device and the mobile device, it is meant that the device and the mobile device are logically connected such that data originating from the first and second sensors are accessible to the mobile device. Any convenient protocol may be implemented to connect the device and the mobile device. For example, in certain embodiments, a wire or series of wires, i.e., a bus, may operably connect the device and the mobile device. Alternatively, a wireless connection, including a Bluetooth connection, may operably connect the device and the mobile device.

The mobile device may be any convenient mobile device. While the nature of the mobile device may vary, e.g., as described herein, in some instances the mobile device is a tablet or a smart phone. The mobile device may be a commercially available, "off-the-shelf" mobile device. For example, the mobile device could be, for example, an Apple iPhone or a Samsung Galaxy phone.

**In** certain embodiments of the system, the wearable device component of the system may also include a third sensor configured to sense head movement, position and/or orientation of the subject. In such embodiments, the wearable device and the mobile device are operably coupled such that data originating from the first, second and third sensors are accessible to the mobile device. The wearable device and the mobile device may be operably coupled as described above. In certain embodiments of the system, the device component of the system may also include a photosensor configured to sense ambient light in the vicinity of the subject. In such embodiments, the device and the mobile device are operably coupled such that data originating from the first, second and third sensors and the photosensor are accessible to the mobile device. The device and the mobile device may be operably coupled as described above.

**In** some embodiments, the processor, the memory and the instructions stored thereon are configured to apply an algorithm to the data that originated from the sensors. In some embodiments, the algorithm applied to the data may be an algorithm for classifying different eye movements, distinguishing between horizontal, vertical and torsional eye movements, recognizing one or more specific patterns of eye movement sensor data comprising one or more characteristic eye movements, including, for example, characteristic eye movements corresponding to neutral gaze, leftward gaze, rightward gaze, upward gaze and downward gaze, or characteristic eye movements corresponding a nystagmus event. In some embodiments, the algorithm applied to the data as described herein may be a machine learning algorithm.

**FIGS. 5A-5F** illustrate a processing algorithm and its constituent steps that may be applied to a signal detected by a wearable device of a system, according to some embodiments. **FIG. 5A** shows the steps of an illustrative algorithm **500** as they are applied to a recorded electronystagmography (ENG) signal **505** that originated from the sensors, according to one embodiment. In step **510,** a noise filter is applied to the signal to filter out noise. For example a 60 Hz noise filter can be applied to the signal to filter out 60 Hz noise. A digital bandpass filter may be applied to filter out signals not between 0.1 to 100 Hz to avoid unwanted high frequency coupling. By way of example, if the desired eye movement signal is close to 60 Hz, a 60Hz digital notch filter may be applied to filter out environmental noise. In some embodiments, after filters are applied to the signal that originated from the sensors, the signal may be referred to as "cleaned."

In step **515,** the cleaned signal may be scaled and/or biased. For example, the signal may be biased to 0 to facilitate later processing. DC electrode offset cancellation may be required for sensing signals in a biological context for signals below 100 Hz. Accordingly, in some instances, DC electrode offset cancellation may also be applied to the signal in step **515.**

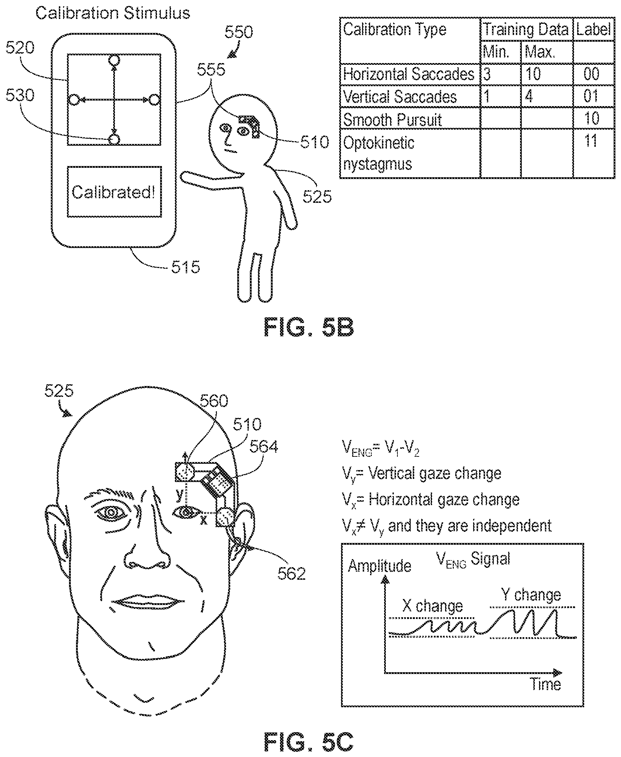

After preprocessing, in step **520,** parameters used in connection with the algorithm are calibrated as the user follows instructions displayed on the mobile device on which the algorithm is downloaded or stored. Such calibration data, obtained as the user follows the instructions displayed on the mobile device, is saved and labeled. The calibration process is described further below, with respect to **FIG. 5B.** The calibration data may be used for later training of more specialized algorithms, including artificial intelligence algorithms, capable of classifying specific types of eye movements. The calibration data may also be used to condition, calibrate or train one or more independent signal extraction algorithms, for example but not limited to, general independent signal extraction algorithms and independent component analysis (ICA) algorithm to conduct independent component analysis separation. In some instances, ICA separation is conducted using a machine learning algorithm. ICA separation may be used to "unmix" the distinct horizontal and vertical motion of a subject's eye using only the collected signal **505,** i.e., the observed ENG signal. After calibrating, in step **525,** ICA separation, using the calibration data, is conducted on the signal to isolate distinct horizontal and vertical motion of the subject's eye. ICA separation is described further below.

In one example, after conducting ICA separation, a pattern recognition step **530** is applied, wherein patterns of horizontal and vertical eye movement that correspond to nystagmus events may be identified. After pattern recognition, a classification step **535** is applied, wherein patterns of eye movements are classified. For example, in some cases, the algorithm may classify patterns of eye movements as those associated with benign paroxysmal positioning vertigo. In some cases, the algorithm may classify patterns of eye movements as those associated with Menière's disease. In some cases, the algorithm may classify patterns of eye movements as those associated with vestibular neuritis may be identified. In some instances, based on the results of the pattern recognition step, the algorithm may determine that the processed signal is associated with a dizziness attack, such as a vertigo attack **540.**

The calibration process, mentioned above, is further illustrated in **FIG. 5B.** An example of the calibration process **550** in connection with a system **555** comprising a wearable device **510** and a software application installed on a mobile device **515.** As seen, instructions **520** are displayed on an output screen of mobile device **515.** Instructions **520** guide a subject **525** to track dots, such as dot **530** which is representative, with his or her eyes as the dots are displayed on the output screen of mobile device **515.** In some cases, a single dot at a time may be displayed on mobile device **515** and may move in horizontal and/or vertical directions. In some instances, the single dot may move exclusively in a horizontal direction followed by moving exclusively in a vertical direction, or vice versa. In some instances, the dot may be moved to elicit torsional eye movement by the subject. The speed at which a dot moves may be any convenient speed and may vary. The mobile device may be held a fixed distance away from the subject's eyes when applying the calibration process. For example, the mobile device may need to be held 30 cm or more away from the face, such as 60 cm. In some instances, the mobile device must be held at a nearly constant distance from the subject's face. In some instances, the instructions guiding the subject may direct the subject to move the mobile device further away or closer to the subject's face. Typically, the mobile device guides the subject to hold the mobile device in a fixed upright orientation. In some instances, the instructions guiding the subject may direct the subject to move or rotate the mobile device into an upright orientation.

**FIG. 5C** illustrates certain characteristics of the observed ENG signal **505** in response to different characteristic eye movements by subject **525**. The wearable device **510** component of a system includes first and second sensors, **560, 562,** and circuitry **564.** Wearable device **510** detects a voltage difference between two electrodes of sensors **560, 562,** positioned proximal to the eye of subject **525** and is applied to a single side of the subject's face. Since eyes have different gaze ranges in the horizontal and vertical directions, the observed signal **505** in each of the horizontal and vertical directions exhibits different amplitudes, as seen in the inset graph of FIG. 5C.

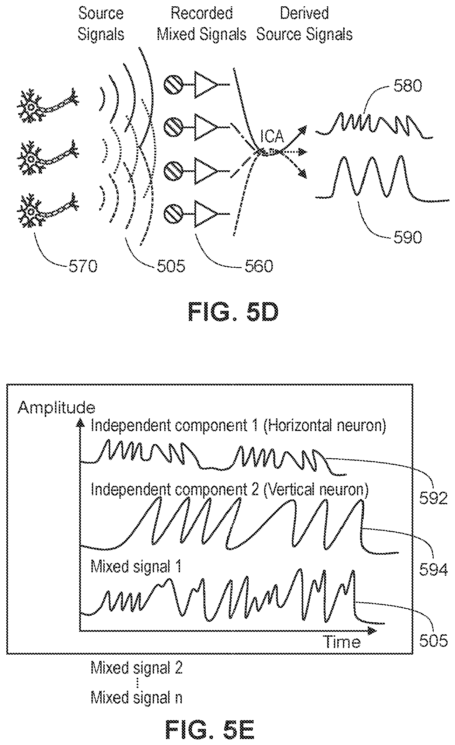

**FIG. 5D** illustrates how a signal **505** measured by sensors **560** of a wearable device, generated by electrical activity in the underlying tissue **570** - is a linear mixing of two signals. Specifically, the signal measured by sensors **560** is a linear mixing of a signal corresponding to horizontal movement **580** of the subject's eye and a signal corresponding to vertical movement **590** of the subject's eye. **FIG. 5D** illustrates that ICA separation is applied to signal **505** measured by the sensors (the observed ENG signal) in order to "unmix" or isolate a signal **580** corresponding to horizontal movement of the subject's eye and a signal **590** corresponding to vertical movement of the subject's eye.

**FIG. 5E** illustrates how the signal **505** measured by sensors is a linear mixing of two signals - a signal corresponding to horizontal movement of the eye (line **592**) and a signal corresponding to vertical movement of the eye (line **594**). Since the signal measured by sensors is a linear combination of horizontal and vertical eye movement components, as shown in **FIGS. 5D-5E,** independent component analysis (ICA) may be used to derive - *i.e.,* "unmix" - the independent source signals corresponding to vertical and horizontal eye movement signals from the recorded mixed signal. Alternatively, in certain embodiments, multiplexing through pairs of vertically and horizontally aligned electrodes may also decouple - *i.e.,* "unmix" - the independent source signals corresponding to vertical and horizontal eye movement signals from the recorded mixed signal. Independent component analysis is described further below.

ICA separation may be used to "unmix" signals corresponding to distinct horizontal and vertical motion of the subject's eye using only the collected signal, i.e., the observed ENG signal. As illustrated above and in **FIGS. 5D-5E,** horizontal eye movement and vertical eye movement are independent components of the observed ENG signal. That is, the observed ENG signal is a linear combination of a signal corresponding to horizontal movement and a signal corresponding to vertical movement. Therefore, the observed ENG signal that is detected by the sensors of the device may be written in the following mathematical notation:

$$x = As$$

Where, in the above, the vector x represents the recorded signals from the sensors; the vector s represents source signals (i.e., distinct signals corresponding to vertical and horizontal components of eye motion), and the matrix A is a "mixing" matrix. To reconstruct the source signals, ICA separation may be applied. ICA is an algorithm for quickly finding the inverse of the matrix A. The inverse of matrix A is called the "unmixing" matrix and is commonly denoted as W. That is:

$$W = A^{-1}$$

Therefore, it follows that:

$$s = Wx$$

Thus, once the "unmixing" matrix, W, is computed, reconstructing the source signal, *s* (i.e., the distinct signals corresponding to vertical and horizontal components of eye motion) is a matter of matrix multiplication between the "unmixing" matrix, *W,* and the recorded signals, *x.*

The ICA algorithm may be implemented in hardware or software in order to be applied to signals detected by sensors, as described herein. With respect to software implementations, any convenient software implementation may be applied. For example, open source implementations of the ICA algorithm, such as Python's scikit-learn, may be applied as convenient.

In certain embodiments, the calibration data described above may be updated through manual label updating. **FIG. 5F** illustrates an exemplary manual label updating process **595.** After calibration, a basic training dataset **596** has been established according to extraction of user's gaze range. The training dataset will be used as reference **597** for comparison **598** with observed signals that are outlier signals **599.** If the outlier signal **599** is larger than the reference value **597,** the ML updating process triggers an update of new parameters for the "unmixing" matrix, *W*, as described above. Also, an update flag may be sent to the user interface for confirming the correctness of the event associated with the outlier signal **599.** In some cases, the subject can also manually update labels associated with events in the training dataset **596.** Typically, the accuracy of this manual label updating process improves with additional recorded training data and labels.

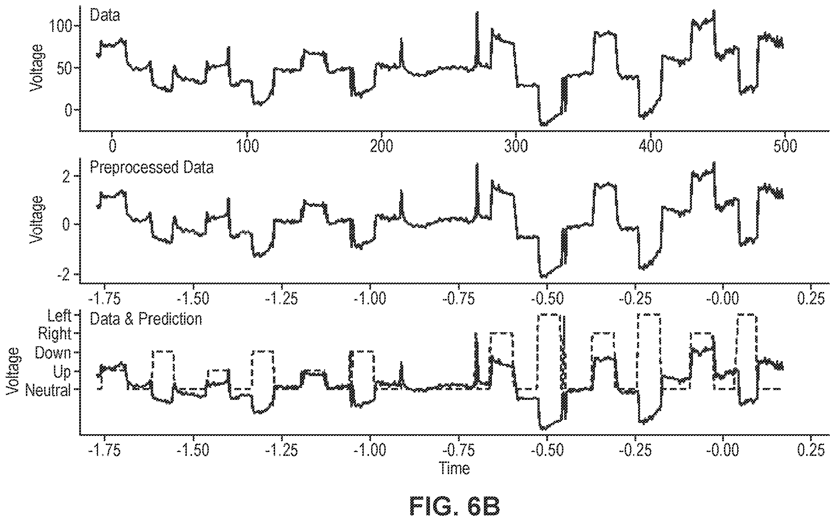

Since dizziness events corresponding to different causes, such as benign paroxysmal positioning vertigo, vestibular migraines and Meniere's disease, are associated with different signal patterns (e.g., see **FIG. 5A**), additional pattern recognition and classification steps may be applied to the signal. The pattern recognition and classification steps are applied to recognize eye and, in some instances, head movement, position and/or orientation patterns by interpreting voltage amplitude and frequency of recorded signals. **FIG. 6A** provides a view of a wearable device **600** component of a system and its use in connection with a subject **610.** Device **600** comprises first and second sensors, a third sensor and circuitry. The first and second sensors are configured to sense eye movement of the subject. The third sensor is configured to sense head movement, position and/or orientation of the subject. Device **600** also comprises a transmitter that is a wireless transmitter **620. FIG. 6A** shows exemplary voltage amplitude and frequency patterns resulting from eye movements **630** and head movement, position and/or orientation **640.** The system may be configured to offer diagnostic suggestions **650** based on recognizing certain patterns of characteristic eye and head movement, position and/or orientation, for example those associated with nystagmus events, such as nystagmus events associated with benign paroxysmal positioning vertigo or nystagmus events associated with Menière's disease or nystagmus events associated with vestibular neuritis. **FIG. 6B** shows an example of classifying an observed ENG signal of a subject into five different categories corresponding to a subject looking left, right, down or up or maintaining a neutral gaze. The upper panel in **FIG. 6B** shows raw data from the wearable device. The middle panel in **FIG. 6B** shows the preprocessed data and the lower panel shows the data after signal process using an algorithm, to make eye movement direction and amplitude easier to read.

As additional data are collected, algorithms applied to detected signals may be extended to use machine learning to classify not only characteristic eye motion, but also the underlying diseases themselves. For example, Long Short Term Models (LSTMs) are a type of recurrent neural network that have been shown to robustly classify time series data such as wearable ECG device data for heart disease. By applying LSTMs to signal data collected according to the present invention, disease diagnoses may be recommended to physicians to increase treatment rate.

The system may include an additional storage component. By additional storage component, it is meant an electronic memory device capable of reading and writing signal information measured by the device as well as related data. Typically, the additional storage component is a commercially available, "off-the-shelf" memory unit. For example, in different embodiments, additional storage component may be an electronic memory device such as an external hard drive, a flash memory drive, an SD card, or the like. The additional storage component is operably coupled to the processor and memory with instructions thereon, such that the processor, the memory and the instructions stored thereon are configured to record data onto the additional storage component. For example, in different embodiments of the system, the processor, the memory and the instructions stored thereon may be configured to record data originating from the first and second sensors onto the additional storage component, or to record data originating from the first, second and third sensors onto the additional storage component, or to record data originating from the first, second and third sensors and the photosensor onto the additional storage component.

In some embodiments, the mobile device may further comprise a display. For example, the mobile device may include a digital readout, screen, monitor, etc. When the mobile device further comprises a display, the processor, the memory and the instructions stored thereon may be configured to display a graphical representation of data onto the display, in a graphical user interface (GUI) etc. For example, in different embodiments of the system, originating from the first and second sensors onto the display on the mobile device, the processor, the memory and the instructions stored thereon are configured to display a graphical representation of the data originating from the first and second sensors onto the display, or to display a graphical representation of the data originating from the first, second and third sensors onto the display, or to display a graphical representation of the data originating from the first, second and third sensors and the photosensor onto the display. The system may be configured to display a graphical representation of the data onto the display in near real time.

In another embodiment, the system comprises a wearable device comprising first and second sensors configured to sense eye movement of the subject and a transmitter configured to transmit signals sensed by the first and second sensors to remote circuitry configured to receive signals transmitted by the transmitter and to detect horizontal and vertical eye movement based on signals from the first and second sensors, and a mobile device comprising a processor operably coupled to a memory that includes instructions stored thereon for interfacing with the device as well as an additional storage component, wherein the device and the mobile device are operably coupled such that data originating from the first and second sensors are accessible to a mobile device. A software application comprising an algorithm for processing signal transmitted from the wearable device is installed on the mobile device.

Additionally, in certain embodiments, the device component of the system may also include a third sensor configured to sense head movement, position and/or orientation of the subject. In such embodiments, the transmitter is further configured to transmit signals sensed by the third sensor to remote circuitry configured to receive signals from the transmitter and detect head movement, position and/or orientation by the subject based on signals received from the transmitter.

Additionally, in some embodiments, the device component of the system may also include a photosensor configured to sense ambient light in the vicinity of the subject. In such embodiments, the transmitter is further configured to transmit signals sensed by the photosensor to remote circuitry configured to receive signals from the photosensor and detect ambient light in the vicinity of the subject based on signals received from the transmitter.

In embodiments of the system wherein the processor, the memory and the instructions stored thereon are configured to apply an algorithm to the data to recognize characteristic eye movements corresponding a nystagmus event, the system may be further configured to recognize a nystagmus event in near real time. In such embodiments, the system may be further configured to distinguish between characteristic eye movements corresponding to horizontal nystagmus events, vertical nystagmus events and torsional nystagmus events. In other embodiments, the system may be configured to recognize characteristic eye movements of nystagmus events associated with benign paroxysmal positional vertigo, or to recognize characteristic eye movements of nystagmus events associated with Menière's disease, or to recognize characteristic eye movements of nystagmus events associated with vestibular neuritis.

An exemplary system is illustrated in **FIG. 7**. A system **700** for monitoring eye movement of a subject comprises a wearable device **710** comprising first and second sensors, such as representative sensor **720,** and circuitry **730.** The system also comprises a software application downloadable to, or as illustrated, downloaded onto a mobile device **740.** The first and second sensors are configured to sense electrical signal associated with eye movement of the subject. In the system illustrated in **FIG. 7,** the first and second sensors are surface electrodes. Circuitry **730** is insertable, preferably removably insertable, into device **710,** for operably connection to sensor **720.** The mobile device **740** comprises a processor operably coupled to a memory that includes instructions stored thereon for interfacing with the wearable device **710** as well as an additional storage component. The processor, memory and instructions stored thereon of the mobile device **740** of the system **700** may be configured to apply an algorithm to the data detected by the sensor **720** to recognize characteristic eye movements, such as nystagmus events. For example, the processor, memory and instructions stored thereon of the system may be configured to apply an algorithm to recognize nystagmus events associated with benign paroxysmal positioning vertigo or nystagmus events associated with Menière's disease or nystagmus events associated with vestibular neuritis. The algorithm applied to the data detected by the sensors may be a machine learning algorithm.

In some embodiments of the device, the algorithm is configured to detect torsional eye movements. In some embodiments, the algorithm is further configured to distinguish between horizontal, vertical and torsional eye movements, or to recognize one or more specific patterns of eye movement sensor data comprising one or more characteristic eye movements, including, for example, recognizing neutral gaze, leftward gaze, rightward gaze, upward gaze and downward gaze, or, in other embodiments, recognizing a nystagmus event. The algorithm may be configured to recognize a nystagmus event in near real time. In other embodiments, the algorithm may be configured to distinguish between characteristic eye movements of horizontal nystagmus events, vertical nystagmus events and/or torsional nystagmus events. The algorithm may be further configured to recognize characteristic eye movements of nystagmus events associated with benign paroxysmal positioning vertigo, or to recognize characteristic eye movements of nystagmus events associated with Menière's disease, or to recognize characteristic eye movements of nystagmus events associated with vestibular neuritis.

The various algorithm steps described in connection with the embodiments disclosed herein can be implemented as electronic hardware, computer software, or combinations of both. To clearly illustrate this interchangeability of hardware and software, various illustrative steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative steps, components, and computing systems described in connection with the embodiments disclosed herein can be implemented or performed by a machine, such as a general purpose processor, a graphics processor unit, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor can also include primarily analog components. A computing environment can include any type of computer system, including, but not limited to, a computer system based on a microprocessor, a graphics processor unit, a mainframe computer, a digital signal processor, a portable computing device, a personal organizer, a device controller, and a computational engine within an appliance, to name a few.

The steps of a method, process, or algorithm, and database used in said steps, described in connection with the embodiments disclosed herein can be embodied directly in hardware, in a software module executed by a processor, or in a combination of the two. A software module, engine, and associated databases can reside in memory resources such as in RAM memory, FRAM memory, flash memory, ROM memory, EPROM memory, EEPROM memory, registers, hard disk, a removable disk, a CD-ROM, or any other form of non-transitory computer-readable storage medium, media, or physical computer storage known in the art. An exemplary storage medium can be coupled to the processor such that the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

Based on the foregoing, it is appreciated that a wearable device is contemplated. The device comprises a sensor configured to provide output data corresponding to eye movement by a user wearing the device, and a control logic comprising instructions for (i) retrieving and/or receiving or obtaining the output data from the sensor, wherein the output contains information about the user's eye movements; and (ii) decorrelation or demixing of the output data into data indicative of horizontal eye movement and/or data indicative of vertical eye movement to create a diagnostic profile of signal; and (iii) conveying the diagnostic profile for diagnosis. In an embodiment, the output data is sensitive to head motion of the user, and the control logic comprises an algorithm to account for or exclude head motion. In an embodiment, the diagnostic profile is based on signal from the wearable device processed by an algorithm as described herein.

Also as described herein, it will be appreciated that in one embodiment, the wearable device transmits a mixed signal of corneo-retinal potential (CRP) data to a computing device that analyses the mixed signal to separate signal arising from horizontal CRP potential and vertical CRP potential from the mixed signal data set, and using one or both to confirm a dizzy episode and/or diagnose cause of the dizzy episode.

Another embodiment of the system comprises a wearable device and a software application, where the software application resides on a computing device and is configured to interact with the wearable device to (i) provide instructions/feedback to a user of the wearable device (e.g., loss of adherence or failure; successful transmission of apparent vertigo event); (ii) analyses of data collected from the device and/or transmission of the raw data from the wearable device or analysed data, to a medical provider; and/or (iii) generation of a report to classify a vertigo event and/or clinically significant indicative eye motions and/or likelihood of underlying conditions. In one embodiment, a camera on the computing device is used to provide feedback about proper placement of the wearable device on the fact. In an embodiment, raw or analysed data from the system is transmitted to a centralized storage and analysis computing system.

II. Methods of Use

Also provided (however, not falling within the scope of the claims by themselves) are methods of detecting horizontal and vertical eye movements of a subject. The methods comprise sensing electrical activity of the subject's eye at a first location; sensing electrical activity of the subject's eye at a second location; and measuring electrical signals correlated with eye movement based on the electrical activity sensed at the first and second locations. The horizontal and vertical eye movements of the subject may be detected in different ways, e.g., measuring the difference in electrical potential between the subject's cornea and retina based on the electrical activity sensed at the first and second locations on the subject. Alternatively, the horizontal and vertical eye movements of the subject may be detected by measuring electrical activity of muscles sensed at the first and second locations on the subject.

In another embodiment, a method for diagnosing cause of episodic dizziness is provided. The method comprises providing a wearable device, and instructing to place or placing the device on a subject at risk of episodic dizziness or experiencing episodic dizziness.

In another embodiment, a method for monitoring electrical data associated with CRP activity is provided. The method comprises applying a wearable device to facial skin of a person, the device comprising a single unitary adhesive assembly comprising a hardware processer and two electrodes configured to detect or derive signal from CRP activity; storing signal detected or derived from the electrodes in the processor; wirelessly transmitting the signal to a computing system; and analyzing the signal to ascertain a baseline of CRP activity and to search for CRP activity connected to an episode of dizziness.

In another embodiment, a method for diagnosing cause of episodic dizziness is provided. The method comprises applying a wearable device to the user's face; the device comprising of two or more electrodes configured such that all electrodes are contained within the area bounded by the sagittal plane passing through the center of face and the transverse plane passing through the bottom of the nose; storing signal detected or derived from the electrodes in the processor; and analyzing the signal to ascertain a baseline of CRP activity and to search for CRP activity connected to an episode of dizziness. Alternatively, the method comprises providing a wearable device as described herein, and instructing to place or placing the device on a subject at risk of episodic dizziness or experiencing episodic dizziness. In one embodiment, the device captures signal correlated with eye movement of a user during an episodic dizzy attack, thereby improving accurate diagnosis of causation.

In another embodiment, a method for evaluating vestibular function is provided. The method comprises applying a wearable device to a user's face; the device comprising two or more electrodes configured to be contained within an area bounded by a sagittal plane passing through the center of the face and a transverse plane passing through a bottom of a nose on the face; storing signal detected or derived from the electrodes in the processor; and decorrelating the signal from a pair of electrodes into the signal related to horizontal and vertical components of the CRP.

In another embodiment, a method of analyzing CRP information is provided. The method comprises collecting information from a wearable device with two electrodes configured to detect or derive signal from CRP activity, the information comprising normal CRP activity and episodic dizziness CRP activity, decorrelating the signal from two or more electrodes into the signal related to horizontal and vertical signals, analyzing the decorrelated signal to generate a report, and optionally providing the report or a diagnostic to a user.

In another embodiment, a method monitoring electrical data associated with CRP activity is provided. The method comprises applying a wearable device to facial skin of a person, the device comprising a single unitary adhesive assembly comprising a hardware processer and at least two electrodes configured to capture CRP activity arising from ocular motion; storing signal detected or derived from the electrodes; processing gathered signals on-board the device; wirelessly transmitting the signal to a computing system; and analyzing the signal to ascertain a Physician interpretable readout of CRP signals and head motion and to search for CRP activity connected to an episode of dizziness.

The methods may also include sensing the acceleration of the subject's head at a third location on the subject and measuring the movement of the subject's head based on the acceleration sensed at the third location. The method may further include sensing ambient light and measuring ambient light based on the ambient light sensed.

The horizontal and vertical eye movements of a subject may be detected by measuring electrical activity of the subject's eye at first and second locations by a wearable device with first and second sensors configured to sense eye movement of the subject and circuitry operably coupled to the sensors and configured to detect horizontal and vertical eye movements based on signals from the first and second sensors. That is, the first and second sensors may be integrated with the circuitry used to process signals from the sensors. In other instances, the movement of the subject's head may be detected by sensing the acceleration of the subject's head at a third location by the wearable device further comprising a third sensor configured to sense head movement, position and/or orientation of the subject and wherein the circuitry is operably connected to the third sensor and configured to detect head movement, position and/or orientation of the subject based on signals from the third sensor. In still other instances, ambient light may be detected by sensing ambient light by the wearable device further comprising a photosensor configured to sense ambient light and wherein the circuitry is configured to detect ambient light based on signals from the photosensor.

Alternatively, horizontal and vertical eye movements of a subject may be detected by measuring electrical activity of the subject's eye at first and second locations by a system comprising a device with first and second sensors configured to sense eye movement of the subject and a transmitter configured to transmit signals sensed by the first and second sensors to remote processing software, such as an algorithm, configured to detect horizontal and vertical eye movement based on signals from the first and second sensors, and a mobile device. That is, the circuitry and/or the algorithm used to process signals from the first and second sensors may be remote from the sensors. In other instances, the movement of the subject's head may be detected by sensing the acceleration of the subject's head at a third location by the foregoing system wherein the constituent device further comprises a third sensor configured to sense head movement, position and/or orientation of the subject and wherein the transmitter is further configured to transmit signals sensed by the third sensor to the remote circuitry that is further configured to detect head movement, position and/or orientation based on signals from the third sensor. In still other instances, ambient light may be detected by sensing ambient light by the foregoing system wherein the constituent device further comprises a photosensor configured to sense ambient light and wherein the transmitter is further configured to transmit signals sensed by photosensor to the remote circuitry that is further configured to detect ambient light based on signals from the photosensor.

The devices, systems and methods may be employed in any application where detecting horizontal and vertical eye movement of a subject is desired. In certain instances, the devices, systems and methods find use detecting head movement, position and/or orientation of a subject or ambient light in the proximity of the subject while simultaneously detecting horizontal and vertical eye movement of the subject.

In some embodiments, the device or system comprises an event trigger mechanism, such as a button or switch on the device or an electronic button presented by the software application, for the patient to activate and/or deactivate the device. The event trigger may encode the recorded data that allows a reader of the data to recognize the beginning and end of a patient reported attack.

In some instances, the devices, systems and methods are employed in the diagnosis and treatment of subjects who experience dizziness or dizzy spells. In other instances, the devices, systems and methods may be employed in the diagnosis of nystagmus events, such as, for example detecting horizontal nystagmus events, vertical nystagmus events or torsional nystagmus events. For example, instances of the device may be used to diagnose benign paroxysmal positioning vertigo by recognizing characteristic eye movements of nystagmus events associated with benign paroxysmal positioning vertigo; instances of the device may be used to diagnose Menière's disease by recognizing characteristic eye movements of nystagmus events associated with Menière's disease; or instances of the device may be used to diagnose vestibular neuritis by recognizing characteristic eye movements of nystagmus events associated with vestibular neuritis.

In some instances, the devices, systems and methods are employed to measure nystagmus events that occur outside the clinical setting, such as when the subject is at home or at work.

In other embodiments, the devices and systems are used to detect a vestibular disorder or a neurological disorders that can impact the vestibule-ocular reflex, smooth pursuit, gaze, and/or saccadic eye movements. In another embodiment, the devices and systems are used in a person with a traumatic brain injury.

Also provided are kits that include at least one or more wearable devices, e.g., as described above. In some instances, a kit may include the parts of the device or disparate components of a system. The kit components may be present in packaging, which packaging may be sterile, as desired.

Also present in the kit may be instructions for using the kit components. The instructions may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging), etc. In other embodiments, the instructions are present as an electronic storage data file present on a suitable computer readable storage medium, e.g., portable flash drive, DVD- or CD-ROM, etc. In other embodiments, the instructions are accessible at a given website address where they can be viewed and/or downloaded by a user. The instructions may take any form, including complete instructions for how to use the device or to troubleshoot the device.

Accordingly, in one embodiment, a kit for monitoring eye movement of a subject comprises a wearable device for monitoring eye movement of a subject and configured to be applied to a single side of a subject's face during use, as described herein. The device comprises first and second sensors configured to sense eye movement of the subject; and circuitry operably coupled to the sensors and configured to detect horizontal and vertical eye movements based on signals from the first and second sensors; and packaging for the device.

In one embodiment, the device of the kit further comprises a third sensor configured to sense head movement, position and/or orientation, wherein the circuitry is operably coupled to the third sensor and is further configured to detect head movement, position and/or orientation based on signals from the third sensor.

In one embodiment, the device of the kit further comprises a photosensor configured to sense ambient light, wherein the circuitry is operably coupled to the photosensor and is configured to detect ambient light based on signals from the photosensor.

In one embodiment, the device of the kit further comprises a storage component operably coupled to the circuitry, wherein the circuitry and the storage component are configured to record eye movement data and head movement, position and/or orientation data onto the storage component. In one embodiment, the circuitry is further configured to recognize one or more specific patterns of eye movement sensor data comprising a nystagmus event.

In another embodiment, a kit for monitoring eye movement of a subject comprises a wearable device for monitoring eye movement of a subject and configured to be applied to a single side of a subject's face during use. The device comprises first and second sensors configured to sense eye movement of the subject and a transmitter configured to transmit signals sensed by the first and second sensors to remote circuitry configured to receive signals transmitted by the transmitter and to detect horizontal and vertical eye movement based on signals from the first and second sensors, and packaging for the device.

The devices, systems, methods and kits described herein are for monitoring eye movement of a subject. As such, devices, systems, methods and kits are provided for detecting horizontal and vertical eye movements of a subject and distinguishing therebetween. In some cases, the devices, systems, methods and kits provided may be used to detect horizontal, vertical and torsional eye movements and to distinguish therebetween. In some cases, the devices, systems, methods and kits provided may be used to detect head movement, position and/or orientation and ambient light in the vicinity of the subject. As such, the devices, systems, methods and kits may facilitate diagnosis of various conditions associated with dizziness attacks, such as benign paroxysmal positioning vertigo, Meniere's disease and vestibular migraines in subjects. The subject is generally a human subject, may be male or female, and may be any age.

A prototype wearable device was developed for measuring horizontal and vertical eye movements of a subject. The prototype was evaluated by comparing against a video electronystagmography (VNG) system such as those currently used in clinical settings to evaluate dizziness attacks in order to determine whether the prototype wearable device could at least replicate the same recordings as the "gold standard" VNG in tracking eye movements. A direct comparison was made by applying VNG goggles and the prototype to patients at the same time and recording eye movements via both the VNG goggles and the prototype wearable sensor.

**FIG. 8** depicts the prototype wearable device for monitoring eye movement of a subject. Wearable device **800** for monitoring eye movement of a subject comprises first and second sensors, indicated collectively by **810,** and circuitry **820.** The first and second sensors are configured to sense eye movement of the subject, as described in detail above. The first and second sensors in this embodiment are one or more first and second electrodes, where the electrodes are surface electrodes that are dry electrodes. As discussed in detail above, the algorithm is configured to detect horizontal and vertical eye movements based on signals from the first and second sensors.

**FIGS. 9A-9C** depicts a prototype wearable device **900** for monitoring eye movement of a subject. The device is comprised of a single wearable patch that is adhered to a facial location of the subject. The wearable device comprises first and second sensors, indicated collectively by **910,** and circuitry **920. FIG. 9A** shows a view of the device when a subject is facing forward. **FIG. 9B** shows a view of the device when a subject is facing slightly forward and slightly to the side. **FIG. 9C** shows a view of the device when a subject is facing to the side.

In an embodiment, the device or the system additionally comprises a mechanism that permits a user to activate or deactivate the device. For example, the device can include a button that a user can depress or push to active or deactivate the device. Alternatively, the software application can include an electronic button that the user can touch to activate or deactivate the device. The trigger mechanism permits a user to initiate monitoring of eye movement and to cease monitoring of eye movement, or to attach a label to a data set. For example, a user experiencing a symptom of dizziness or actual dizziness can touch the trigger mechanism to label when the symptom or actual dizziness occurs. The algorithm inspecting the data can look for the label to scrutinize the data in the labeled time frame to determine if dizziness occurred. The trigger mechanism can be touched or activated at the beginning of a perceived dizzy episode and at the end of the episode to bracket the data with labels. The label can take the form of an electrical spike in the data set, that is easily detected by the algorithm. The device or the system can also include an indicator to alert a user of information, such as low battery, circuit failure, on or off status. The indicator can be a light, a sound, a haptic, or the like.

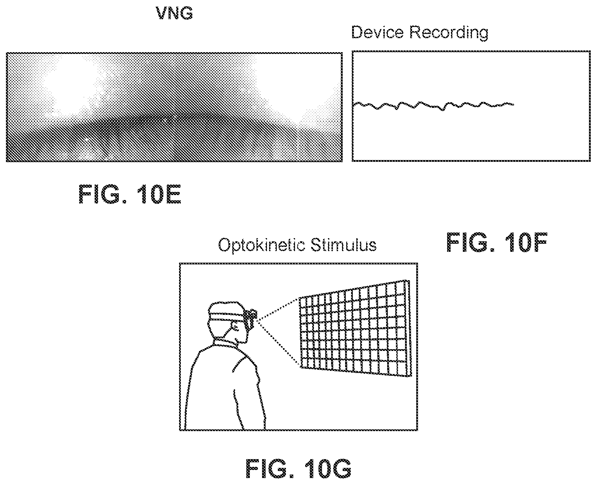

**FIGS. 10A-10G** summarize the results of a direct comparison of video nystagmography (VNG) and the prototype of the wearable sensor described herein to detect and monitor eye movements. In this study, a wearable device like that shown in FIG. 8 was used. Recordings of eye movements using both devices were done under conditions of saccades, smooth pursuit and optokinetics. In **FIGS. 10A-10B**, it is shown that the signal recorded by the prototype wearable device (FIG. 10B, upper and lower graphs "wearable ENG"), ranging from 200 to 800 mV, demonstrates imperviousness to any noise artifact from the infrared VNG goggles. Inter-subject variability in measurements was addressed in nascent stages with testing of five individuals without difficulty in domains of saccades (quick eye movements between two targets), smooth pursuit (smoothly following a target) and with optokinetics (eye movements tracking a repeating target moving horizontally). A checkerboard was used to induce right to left or left to right optokinetic stimulus (**FIG. 10G**) and an sample of the tracing of data signal is shown in **FIG. 10F.** Based on the results obtained, it was found that the prototype wearable device attains equivalent performance to VNG in detecting horizontal eye movements and superior performance to VNG in detecting vertical eye movements (likely due to the fact that VNG is sensitive to eyelid artefacts, as shown in **FIG. 10E**).

**FIGS. 11A-11D** show the results of the direct comparison of downward VNG (**FIG. 11A**) and the prototype wearable device (**FIG. 11B**) in optokinetic recordings. The prototype wearable device exhibited more accurate and reliable recording of vertical eye movements. The wearable device was able to detect torsional eye movements (rotating eye movements). For torsional eye movement recording, the conventional VNG (**FIG. 11C**) was unable to detect vertical torsional change. The inability of VNG to detect vertical torsional change might be caused due to the eyelid artefact discussed above. The prototype wearable device was capable of synchronously record torsional change in both horizontal and vertical channels (**FIG. 11D**). A head movement, position and/or orientation sensor was also incorporated into the prototype device to record eye movements and head movement, position and/or orientation. In some cases, inclusion of a head movement, position and/or orientation sensor can improve diagnostic accuracy. The results of the study conducted on the prototype wearable device showed that it outperforms the clinical gold standard, VNG, for classifying eye movements.

Further, to investigate the acceptability of wearing an ENG device, such as the wearable device described herein, ten patients were interviewed with a prepared survey form. The survey was designed to challenge patient acceptance in various social settings in order to try and elicit information about social embarrassment in connection with wearing the device. The social settings ranged from at home or in bed to dinner with friends or viewing someone wearing the device on television. Survey feedback was positive, particularly for a camouflaged design, similar to a band aid (to which 90% of patients indicated they would be likely to wear).

Another study was conducted using a wearable device as described herein comprised of a unitary substrate with three electrodes. After affixing the device unilaterally to the subject's face, monocular eye movements were detected for 30 seconds, where for the first 15 seconds the subject was asked to make deliberate horizontal eye movement and for the remaining 15 seconds to make deliberate vertical eye movement. **FIG. 12A** shows the raw data from signal detected by each electrode, where each panel (upper panel, middle panel and lower panel) shows the data from one electrode. The raw data, which is a mixed signal from horizontal and vertical eye movement, was processed to remove noise and baseline drift and the processed data for each electrode is shown in the three panels of **FIG. 12B.** The processed data was then analyzed by an algorithm to discriminate or unmix the signal associated with horizontal eye movement from signal associated with vertical eye movement. **FIG. 12C** shows the data after processing by an algorithm, where the upper panel in FIG. 12C shows the signal separated or unmixed to show signal associated with the horizontal eye movement made for the first 15 seconds on of the study, and the lower panel shows the signal associated with the vertical eye movement made during the following 15 seconds. Accordingly, in one embodiment, first and second sensors comprise horizontal and vertical signals derived from three or more electrodes operably coupled with signal processing and extracting algorithms.

There are currently no FDA-approved methods for monitoring dizziness attacks. The current method used by clinicians is video nystagmography (VNG) where infrared eye goggles are worn in clinic. The diagnostic accuracy of VNG/ENG is poor as the majority of patients do not experience an attack in the clinic. There have been some attempts to create at-home versions of VNG diagnostic tools, but they are impractical as they require expensive and cumbersome headsets and require the subject to set up the device during an attack. There are a handful of video-based home monitoring systems that require use of a camera; however, these systems entail obstruction of vision for monitoring. Also, such devices require an attachment and smartphone for use. Moreover, since the apparatus may not be worn around, it must be set up and quickly applied while the subject is having an attack. This makes practical application very difficult for most patients. Another main problem related to video recording methods is that the patient needs to keep his or her eyes open to be monitored by video. This tends to be counter to the patient's desires during the attack as patients prefer to close their eyes when experiencing severe spinning and nausea. Furthermore, limitations are present due to significant artefacts arise from the patient blinking in the ordinary course as well as patient eye makeup. As such, there is a need for wearable, portable devices that can be used for home monitoring such a wearable-ENG, such as the device described herein.

Many variations on the devices, systems, methods and kits described herein will be apparent from this disclosure. For example, depending on the embodiment, certain acts, events, or functions of any of the algorithms described herein can be performed in a different sequence, can be added, merged, or left out altogether (e.g., not all described acts or events are necessary for the practice of the algorithms).

Moreover, in certain embodiments, acts or events can be performed concurrently, e.g., through multi-threaded processing, interrupt processing, or multiple processors or processor cores or on other parallel architectures, rather than sequentially. In addition, different tasks or processes can be performed by different machines and/or computing systems that can function together.

Although the foregoing invention has been described in some detail by way of illustration and example for purposes of clarity of understanding, it is readily apparent to those of ordinary skill in the art in light of the teachings of this invention that certain changes and modifications may be made thereto.

Furthermore, all examples and conditional language recited herein are principally intended to aid the reader in understanding the principles of the invention and the concepts contributed by the inventors to furthering the art and are to be construed as being without limitation to such specifically recited examples and conditions. Moreover, all statements herein reciting principles, aspects, and embodiments as well as specific examples thereof, are intended to encompass both structural and functional equivalents thereof. Additionally, it is intended that such equivalents include both currently known equivalents and equivalents developed in the future, i.e., any elements developed that perform the same function, regardless of structure.

**Claims**

1. A wearable device, comprising:

> a unitary substrate comprising a first electrode and a second electrode; and
> circuitry operably coupled to the first electrode and the second electrode the unitary substrate dimensioned for unilateral placement to only one side of one eye of a user's face to position the first electrode substantially above one eye of the subject and the second electrode substantially to the side of the same eye of the subject, thereby to detect monocular corneo-retinal potentials correlated with horizontal and vertical movement of that single eye,

wherein the circuitry is configured to detect horizontal and vertical movements of that single eye of the user from the corneo-retinal potentials detected by the first electrode and the second electrode.

2. The device according to claim 1, wherein the circuitry comprises an electronic component removably insertable into a compartment on the unitary substrate, and wherein the electronic component when inserted into the compartment is in electronic communication with the circuitry.

3. The device according to claim 1 or claim 2, wherein the circuitry comprises an analog front end and a digital circuit, preferably with one of the following:

   • wherein the analog front end comprises a noise filtering circuit and an amplifier circuit;
   • wherein the digital circuit comprises an analog to digital converter and a microcontroller;
   • wherein the digital circuit further comprises a digital signal processor.

4. The device according to any preceding claim, wherein the first electrode is positioned on the substrate such that when placed on a user's face a midpoint of a plane of the first electrode is superior to a transverse (horizontal) plane passing through the center of one of the right eye or the left eye, and the second electrode is positioned on the substrate such that when placed on a user's face a midpoint of a plane of the second electrode is positioned temporally to a sagittal plane passing through a pupil of the eye when looking straight ahead.

5. The device according to any preceding claim, further comprising a third sensor, wherein the third sensor is configured to sense head position, head movement, and/or head orientation of the user, and wherein the circuitry is operably coupled to the third sensor, preferably wherein the third sensor is an accelerometer, an inertial mass unit, a magnetometer, a gyroscope, or a combination thereof.

6. The device according to claim 5, wherein the device is configured to continuously monitor eye movement and head position, movement or orientation, or wherein the device is configured to monitor eye movement and head position, movement or orientation in near real time.

7. The device according to any of claims 5 or 6, further comprising:

a storage component operably coupled to the circuitry, wherein the circuitry and the storage component are configured to record eye movement data and head position, movement or orientation data onto the storage component, preferably wherein the storage component is a removable memory card.

8. The device according to any of claims 5-7, further comprising a transmitter operably coupled to the circuitry, wherein the circuitry and the transmitter are configured to transmit eye movement data, head movement data, head position data, head orientation data, or a combination thereof, preferably wherein the transmitter is a wireless transmitter and wherein the circuitry, and the wireless transmitter are configured to wirelessly transmit eye movement data and data from head movement, position or orientation.

9. The device according to any preceding claim, with one or more of the following:

   • further comprising a photosensor configured to sense ambient light, wherein the circuitry is operably coupled to the photosensor and is configured to detect ambient light based on signals from the photosensor;
   • wherein the circuitry is further configured to detect torsional eye movements;
   • wherein in addition to the first electrode and a second electrode, the wearable device comprises an electronic component, and the electronic component is on a substrate separate from the unitary substrate on which the first electrode and second electrode are affixed;
   • wherein the circuitry comprises an electronic component on a substrate separate from the unitary substrate, and the circuitry is operably coupled to the first electrode and the second electrode.

10. A system for detecting eye movements of a subject, comprising:

a wearable device according to any one of the preceding claims and
a software application comprising an algorithm for processing data received from the wearable device.

11. The system of claim 10 with one or more of the following:

   • wherein the software application is downloadable to a mobile device, preferably
   • wherein the mobile device comprises a processor operably connected to a memory for storage of the algorithm, wherein the algorithm is configured to process the data to (i) distinguish between horizontal, vertical and torsional eye movements or (ii) recognize one or more specific patterns of eye movement, more preferably
• wherein the algorithm process the data to recognize one or more specific patterns of eye movement corresponding to neutral gaze, leftward gaze, rightward gaze, upward gaze, downward gaze or a nystagmus event;
• wherein the system is configured to recognize characteristic eye movements of nystagmus events associated with benign paroxysmal positioning vertigo, Meniere's disease or vestibular neuritis.

**Patentansprüche**

1. Am Körper tragbare Vorrichtung, umfassend:

   ein einheitliches Substrat, das eine erste Elektrode und eine zweite Elektrode umfasst; und Schaltlogik, die betriebsfähig an die erste Elektrode und die zweite Elektrode gekoppelt sind, wobei das einheitliche Substrat für eine einseitige Platzierung an nur einer Seite eines Auges eines Gesichts eines Benutzers dimensioniert ist, um die erste Elektrode im Wesentlichen über einem Auge des Probanden und die zweite Elektrode im Wesentlichen seitlich von demselben Auge des Probanden zu positionieren, um dadurch monokulare korneo-retinale Potenziale zu detektieren, die mit horizontaler und vertikaler Bewegung dieses einzelnen Auges korreliert sind,
   wobei die Schaltlogik konfiguriert ist, um horizontale und vertikale Bewegungen dieses einzelnen Auges des Benutzers aus den korneo-retinalen Potenzialen zu detektieren, die von der ersten Elektrode und der zweiten Elektrode detektiert werden.

2. Vorrichtung nach Anspruch 1, wobei die Schaltlogik eine elektronische Komponente umfasst, die entfernbar in ein Fach auf dem einheitlichen Substrat einsetzbar ist, und wobei die elektronische Komponente, wenn sie in das Fach eingesetzt ist, in elektronischer Kommunikation mit der Schaltlogik steht.

3. Vorrichtung nach Anspruch 1 oder 2, wobei die Schaltlogik ein analoges Front-End und eine digitale Schaltung umfasst, vorzugsweise mit einem der folgenden:

   • wobei das analoge Front-End eine Rauschfilterschaltung und eine Verstärkerschaltung umfasst;

   • wobei die digitale Schaltung einen Analog-Digital-Wandler und einen Mikrocontroller umfasst;
   • wobei die digitale Schaltung ferner einen digitalen Signalprozessor umfasst.

4. Vorrichtung nach einem der vorstehenden Ansprüche, wobei die erste Elektrode auf dem Substrat so positioniert ist, dass bei Platzierung auf dem Gesicht eines Benutzers ein Mittelpunkt einer Ebene der ersten Elektrode oberhalb einer transversalen (horizontalen) Ebene liegt, die durch das Zentrum eines des rechten Auges oder des linken Auges verläuft, und die zweite Elektrode auf dem Substrat so positioniert ist, dass bei Platzierung auf dem Gesicht eines Benutzers ein Mittelpunkt einer Ebene der zweiten Elektrode temporal zu einer sagittalen Ebene positioniert ist, die durch eine Pupille des Auges verläuft, wenn geradeaus geblickt wird.

5. Vorrichtung nach einem der vorstehenden Ansprüche, ferner umfassend einen dritten Sensor, wobei der dritte Sensor konfiguriert ist, um Kopfposition, Kopfbewegung und/oder Kopforientierung des Benutzers zu erfassen, und wobei die Schaltlogik betriebsfähig an den dritten Sensor gekoppelt ist, vorzugsweise wobei der dritte Sensor ein Beschleunigungsmesser, eine Trägheitsmasseneinheit, ein Magnetometer, ein Gyroskop oder eine Kombination davon ist.

6. Vorrichtung nach Anspruch 5, wobei die Vorrichtung konfiguriert ist, um Augenbewegung und Kopfposition, -bewegung oder -orientierung kontinuierlich zu überwachen, oder wobei die Vorrichtung konfiguriert ist, um Augenbewegung und Kopfposition, -bewegung oder -orientierung in nahezu Echtzeit zu überwachen.

7. Vorrichtung nach einem der Ansprüche 5 oder 6, ferner umfassend:
   eine Speicherkomponente, die betriebsfähig an die Schaltlogik gekoppelt ist, wobei die Schaltlogik und die Speicherkomponente konfiguriert sind, um Augenbewegungsdaten und Kopfpositions-, Kopfbewegungs- oder -orientierungsdaten auf der Speicherkomponente aufzuzeichnen, vorzugsweise wobei die Speicherkomponente eine entnehmbare Speicherkarte ist.

8. Vorrichtung nach einem der Ansprüche 5 bis 7, ferner umfassend einen Sender, der betriebsfähig an die Schaltlogik gekoppelt ist, wobei die Schaltlogik und der Sender konfiguriert sind, um Augenbewegungsdaten, Kopfbewegungsdaten, Kopfpositionsdaten, Kopforientierungsdaten oder eine Kombination davon zu übertragen, vorzugsweise wobei der Sender ein drahtloser Sender ist und wobei die Schaltlogik und der drahtlose Sender konfiguriert sind, um Augenbewegungsdaten und Daten von Kopfbewegung, -position oder -orientierung drahtlos zu übertragen.

9. Vorrichtung nach einem der vorstehenden Ansprüche mit einem oder mehreren der folgenden:

• ferner umfassend einen Photosensor, der konfiguriert ist, um Umgebungslicht zu erfassen, wobei die Schaltlogik betriebsfähig an den Photosensor gekoppelt ist und konfiguriert ist, um Umgebungslicht basierend auf Signalen von dem Photosensor zu detektieren;
• wobei die Schaltlogik ferner konfiguriert ist, um torsionale Augenbewegungen zu detektieren;
• wobei zusätzlich zu der ersten Elektrode und einer zweiten Elektrode die am Körper tragbare Vorrichtung eine elektronische Komponente umfasst, und die elektronische Komponente sich auf einem Substrat befindet, das von dem einheitlichen Substrat getrennt ist, auf dem die erste Elektrode und die zweite Elektrode befestigt sind;
• wobei die Schaltlogik eine elektronische Komponente auf einem Substrat getrennt von dem einheitlichen Substrat umfasst, und die Schaltlogik betriebsfähig an die erste Elektrode und die zweite Elektrode gekoppelt ist.

10. System zum Detektieren von Augenbewegungen eines Probanden, umfassend:

eine am Körper tragbare Vorrichtung nach einem der vorstehenden Ansprüche und
eine Softwareanwendung, die einen Algorithmus zur Verarbeitung von Daten, die von der am Körper tragbaren Vorrichtung empfangen werden, umfasst.

11. System nach Anspruch 10 mit einem oder mehreren der folgenden:

• wobei die Softwareanwendung auf eine mobile Vorrichtung herunterladbar ist, vorzugsweise
• wobei die mobile Vorrichtung einen Prozessor umfasst, der betriebsfähig mit einem Speicher zum Speichern des Algorithmus verbunden ist, wobei der Algorithmus konfiguriert ist, um die Daten zu verarbeiten, um (i) zwischen horizontalen, vertikalen und torsionalen Augenbewegungen zu unterscheiden oder (ii) ein oder mehrere spezifische Muster der Augenbewegung zu erkennen, mehr bevorzugt
• wobei der Algorithmus die Daten verarbeitet, um ein oder mehrere spezifische Muster der Augenbewegung zu erkennen, die einem neutralen Blick, einem Blick nach links, einem Blick nach rechts, einem Blick nach oben, einem Blick nach unten oder einem Nystagmus-Ereignis entsprechen;
• wobei das System konfiguriert ist, um charakteristische Augenbewegungen von Nystagmus-Ereignissen zu erkennen, die mit gutartigem paroxysmalen Lagerungsschwindel, Morbus Menière oder Vestibularisneuritis assoziiert sind.

## Revendications

1. Dispositif portable, comprenant :

un substrat unitaire comprenant une première électrode et une seconde électrode ; et un ensemble de circuits couplé de manière opérationnelle à la première électrode et à la seconde électrode, le substrat unitaire étant dimensionné pour un placement unilatéral sur un seul côté d'un œil d'un visage d'un utilisateur pour positionner la première électrode sensiblement au-dessus d'un œil du sujet et la seconde électrode sensiblement sur le côté du même œil du sujet, de ce fait pour détecter des potentiels cornéo-rétiniens monoculaires corrélés au mouvement horizontal et vertical de cet œil unique, dans lequel l'ensemble de circuits est conçu pour détecter des mouvements horizontaux et verticaux de cet œil unique de l'utilisateur à partir des potentiels cornéo-rétiniens détectés par la première électrode et la seconde électrode.

2. Dispositif selon la revendication 1, dans lequel l'ensemble de circuits comprend un composant électronique pouvant être inséré de manière amovible dans un compartiment sur le substrat unitaire, et dans lequel le composant électronique lorsqu'il est inséré dans le compartiment est en communication électronique avec l'ensemble de circuits.

3. Dispositif selon la revendication 1 ou la revendication 2, dans lequel l'ensemble de circuits comprend un frontal analogique et un circuit numérique, de préférence avec l'un des éléments suivants :

• dans lequel le frontal analogique comprend un circuit de filtrage du bruit et un circuit amplificateur ;
• dans lequel le circuit numérique comprend un convertisseur analogique-numérique et un microcontrôleur ;
• dans lequel le circuit numérique comprend en outre un processeur de signal numérique.

4. Dispositif selon une quelconque revendication précédente, dans lequel la première électrode est positionnée sur le substrat de telle sorte que lorsqu'elle est placée sur un visage d'un utilisateur, un point médian d'un plan de la première électrode est supérieur à un plan transversal (horizontal) passant par le centre de l'un parmi l'œil droit ou l'œil gauche, et la seconde électrode est positionnée sur le substrat de telle sorte que lorsqu'elle est placée sur un visage d'un utilisateur, un point médian d'un plan de la seconde électrode est positionné temporellement à un plan sagittal traversant une pupille de l'œil lorsque l'on regarde droit devant.

5. Dispositif selon une quelconque revendication précédente, comprenant en outre un troisième capteur, dans lequel le troisième capteur est conçu pour capter une position de tête, un mouvement de tête et/ou une orientation de tête de l'utilisateur, et dans lequel l'ensemble de circuits est couplé de manière opérationnelle au troisième capteur, de préférence dans lequel le troisième capteur est un accéléromètre, une unité de masse inertielle, un magnétomètre, un gyroscope ou une combinaison de ceux-ci.

6. Dispositif selon la revendication 5, dans lequel le dispositif est conçu pour surveiller en continu un mouvement oculaire et une position, un mouvement ou une orientation de tête, ou dans lequel le dispositif est conçu pour surveiller un mouvement oculaire et une position, un mouvement ou une orientation de tête en temps quasi réel.

7. Dispositif selon l'une quelconque des revendications 5 ou 6, comprenant en outre :
un composant de stockage couplé de manière opérationnelle à l'ensemble de circuits, dans lequel l'ensemble de circuits et le composant de stockage sont conçus pour enregistrer des données de mouvements oculaires et des données de position, de mouvement ou d'orientation de tête sur le composant de stockage, de préférence dans lequel le composant de stockage est une carte mémoire amovible.

8. Dispositif selon l'une quelconque des revendications 5 à 7, comprenant en outre un émetteur couplé de manière opérationnelle à l'ensemble de circuits, dans lequel l'ensemble de circuits et l'émetteur sont conçus pour émettre des données de mouvements oculaires, des données de mouvements de tête, des données de position de tête, des données d'orientation de tête, ou une combinaison de celles-ci, de préférence dans lequel l'émetteur est un émetteur sans fil et dans lequel l'ensemble de circuits et l'émetteur sans fil sont conçus pour émettre sans fil des données de mouvements oculaires et des données de mouvement, de position ou d'orientation de tête.

9. Dispositif selon l'une quelconque revendication précédente, avec un ou plusieurs des éléments suivants :

• comprenant en outre un photocapteur conçu pour capter la lumière ambiante, dans lequel l'ensemble de circuits est couplé de manière opérationnelle au photocapteur et est conçu pour détecter une lumière ambiante en fonction de signaux du photocapteur ;
• dans lequel l'ensemble de circuits est en outre conçu pour détecter des mouvements oculaires de torsion ;
• dans lequel, en plus de la première électrode et d'une seconde électrode, le dispositif portable comprend un composant électronique, et le composant électronique est sur un substrat séparé du substrat unitaire sur lequel la première électrode et la seconde électrode sont fixées ;
• dans lequel l'ensemble de circuits comprend un composant électronique sur un substrat séparé du substrat unitaire, et l'ensemble de circuits est couplé de manière opérationnelle à la première électrode et à la seconde électrode.

10. Système de détection de mouvements oculaires d'un sujet, comprenant :

un dispositif portable selon l'une quelconque des revendications précédentes et
une application logicielle comprenant un algorithme permettant de traiter des données reçues du dispositif portable.

11. Système selon la revendication 10, avec un ou plusieurs des éléments suivants :

• dans lequel l'application logicielle est téléchargeable sur un dispositif mobile, de préférence
• dans lequel le dispositif mobile comprend un processeur connecté de manière opérationnelle à une mémoire pour le stockage de l'algorithme, dans lequel l'algorithme est configuré pour traiter les données pour (i) distinguer entre des mouvements oculaires horizontaux, verticaux et de torsion ou (ii) reconnaître un ou plusieurs modèles spécifiques de mouvements oculaires, plus préférablement
• dans lequel l'algorithme traite les données pour reconnaître un ou plusieurs modèles spécifiques de mouvement oculaire correspondant à un regard neutre, un regard vers la gauche, un regard vers la droite, un regard vers le haut, un regard vers le bas ou un événement de nystagmus ;
• dans lequel le système est conçu pour reconnaître des mouvements oculaires caractéristiques d'événements de nystagmus associés à un vertige positionnel paroxystique bénin, à la maladie de Ménière ou à une névrite vestibulaire.

FIG. 1A

FIG. 1B

FIG. 2A

FIG. 2B

FIG. 2C

FIG. 2D

FIG. 2E

FIG. 2F

FIG. 2G

FIG. 2H

*Prior Art* FIG. 2I

**FIG. 3A**

**FIG. 3B**

**FIG. 4**

FIG. 5A

Calibration Stimulus

| Calibration Type | Training Data | | Label |
|---|---|---|---|
| | Min. | Max. | |
| Horizontal Saccades | 3 | 10 | 00 |
| Vertical Saccades | 1 | 4 | 01 |
| Smooth Pursuit | | | 10 |
| Optokinetic nystagmus | | | 11 |

**FIG. 5B**

$V_{ENG} = V_1 - V_2$

$V_y$ = Vertical gaze change

$V_x$ = Horizontal gaze change

$V_x \neq V_y$ and they are independent

**FIG. 5C**

Source Signals    Recorded Mixed Signals    Derived Source Signals

580

590

570    505    560

**FIG. 5D**

Amplitude

Independent component 1 (Horizontal neuron)

Independent component 2 (Vertical neuron)                    592

Mixed signal 1                    594

Time                    505

Mixed signal 2

Mixed signal n

**FIG. 5E**

595

598

User manual label

599 — Outlier signal

596 — Reference Value and label

597

598

Update flag

ML Updater

**FIG. 5F**

600 620

610

Eye movement

630

Head movement

640

Label

| Normal | Dizzy | Falling | Dizzy | Normal |
|--------|-------|---------|-------|--------|
| 00     | 01    | 11      | 01    | 00     |

24 hours continuous dataset, over 14 days

650

Data storage & analysis

Diagnostic Suggestions

**FIG. 6A**

FIG. 6B

**Circuit**
Flexible & Skin Friendly

**Electrode**
Robust & Disposable

**App**
History and AI based
event alert

Circuit
IP

730  710  720  740

700

**FIG. 7**

800

820

810

**FIG. 8**

FIG. 9A

FIG. 9B

FIG. 9C

VNG

Random Saccade

Horizontal Left

Horizontal Right

Horizontal Left

Horizontal Right

**FIG. 10A**

Wearable ENG

Horizontal Saccade

Smooth Pursuit

**FIG. 10B**

**VNG**

Eye Position [°]  Optokinetic Down 20 dps

**Wearable ENG**

FIG. 10C

FIG. 10D

EP 4 087 465 B1

VNG

Device Recording

**FIG. 10E**

**FIG. 10F**

Optokinetic Stimulus

**FIG. 10G**

VNG

Eye Position [°]   Optokinetic Down 40 dps

Horizontal Left

R

20
15
10
5

1  2  3  4  5  6  7  8  9

-5
-10
-15
-20

L

Horizontal Right

**FIG. 11A**

Wearable ENG

0.5
0.4
0.3
0.2
0.1
-0.0
-0.1
-0.2
-0.3
-0.4
-0.5

Voltage (V)

Optokenetic Down (40dps)

15 16 17 18 19 20 21 22 23 24 25
Time (Second)

**FIG. 11B**

Eye Position [°]
Horizontal                              Horizontal Right

10  R

-10
-20  L                                   Setting

Eye Position [°]
Vertical                                Vertical Right

20  U

10

Can't detect
vertical change

50      55      60      65

10  D              Setting

**FIG. 11C**

Stop   2.000                              0.000000000ps

Horizontal                    Torsion
                              Change

Vertical

**FIG. 11D**

EP 4 087 465 B1

FIG. 12A

FIG. 12B

Unmixed Signals

FIG. 12C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62957563
- EP 338623 A1